(19) Europäisches Patentamt
European Patent Office
Office européen des brevets

(11) Veröffentlichungsnummer: **0 259 782 B1**

(12) # EUROPÄISCHE PATENTSCHRIFT

(45) Veröffentlichungstag der Patentschrift: **27.12.90**

(51) Int. Cl.[5]: **C07C 217/78**, C07C 225/22, C07C 309/01, C07C 311/03, C07C 255/00, C07C 255/58, C07C 211/30, C07C 233/34, A61K 31/135, A61K 31/255, A61K 31/325

(21) Anmeldenummer: **87112865.8**

(22) Anmeldetag: **03.09.87**

(54) **Neue Naphtalin- und Indanderivate, diese Verbindungen enthaltende Arzneimittel und Verfahren zu deren Herstellung.**

(30) Priorität: **11.09.86 DE 3630903**

(43) Veröffentlichungstag der Anmeldung:
**16.03.88 Patentblatt 88/11**

(45) Bekanntmachung des Hinweises auf die Patenterteilung:
**27.12.90 Patentblatt 90/52**

(84) Benannte Vertragsstaaten:
**AT BE CH DE ES FR GB GR IT LI LU NL SE**

(56) Entgegenhaltungen:
**EP-A-0 177 960**
**DE-A-3 324 727**
**DE-B-2 158 619**

**PATENT ABSTRACTS OF JAPAN, unexamined applications, Sektion C, Band 2, Nr. 48, 31. März 1978; THE PATENT OFFICE JAPANESE GOVERNMENT, Seite 128 C 78**

**Die Akte enthält technische Angaben, die nach dem Eingang der Anmeldung eingereicht wurden und die nicht in dieser Patentschrift enthalten sind.**

(73) Patentinhaber: **Dr. Karl Thomae GmbH, Postfach 1755, D-7950 Biberach (Riss)(DE)**

(72) Erfinder: **Psiorz, Manfred, Dr. Dipl.-Chem., Riedlingr Strasse 35, D-7950 Biberach 1(DE)**
Erfinder: **Heider, Joachim, Dr. Dipl.-Chem., Am Hang 3, D-7951 Warthausen 1(DE)**
Erfinder: **Bomhard, Andreas, Dr. Dipl.-Chem., Dinglingerstrasse 9, D-7950 Biberach 1(DE)**
Erfinder: **Hauel, Norbert, Dr. Dipl.-Chem., Stresemannstrasse 40, D-7950 Biberach-Bachlangen(DE)**
Erfinder: **Noll, Klaus, Dr. Dipl.-Chem., Im Schönblick 3, D-7951 Warthausen(DE)**
Erfinder: **Narr, Berthold, Dr. Dipl.-Chem., Obere Au 5, D-7950 Biberach 1(DE)**
Erfinder: **Lillie, Christian, Dr., Hansi-Niese-Weg 12, A-1130 Wien(AT)**
Erfinder: **Kobinger, Walter, Prof Dr., Beighofergasse 27, A-1121 Wien(AT)**
Erfinder: **Dämmgen, Jürgen, Dr., Eichweg 7, D-7951 Sulmingen(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

In der EP-A-0.177.960 werden bereits Tetrahydronaphthaline, welche in 2-Stellung durch eine gegebenenfalls durch einen Acylrest substituierte Hydroxygruppe substituiert sind, beschrieben. Diese Verbindungen besitzen eine ausgeprägte Calcium-antagonistische Wirkung und können deshalb als Arzneimittel verwendet werden, insbesondere für die Bekämpfung bzw. Verhütung von Angina pectoris, Ischämie, Arrhythmien und Bluthochdruck.

Überraschenderweise wurde nun gefunden, daß die neuen Naphthalin- und Indanderivate der Formel

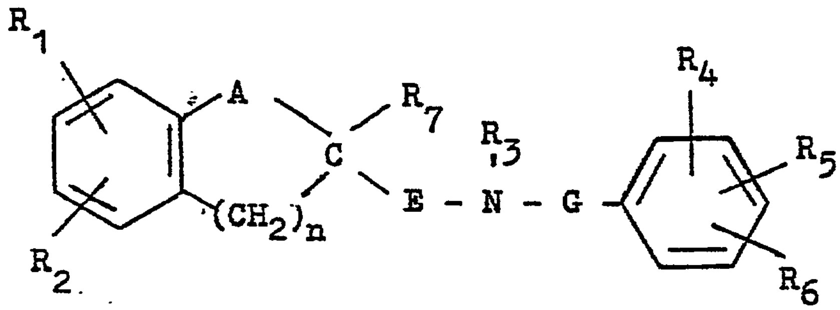

(I)

deren Enantiomeren, deren Diastereomeren und deren Säureadditionssalze, insbesondere deren physiologisch verträgliche Säureadditionssalze mit anorganischen oder organischen Säuren, andere wertvolle pharmakologische Eigenschaften aufweisen, insbesondere eine herzfrequenzsenkende Wirkung und eine herabsetzende Wirkung auf den $O_2$-Bedarf des Herzens.

Gegenstand der vorliegenden Erfindung sind somit die neuen Tetrahydronaphthalin- und Indanderivate der obigen Formel I, deren Enantiomeren, deren Diastereomeren, deren Säureadditionssalze, insbesondere für die pharmazeutische Verwendung deren physiologisch verträgliche Säureadditionssalze mit anorganischen oder organischen Säuren, Verfahren zu ihrer Herstellung und diese Verbindungen enthaltende Arzneimittel.

In der obigen allgemeinen Formel I bedeuten

n die Zahl 1 oder 2,

A eine Carbonylgruppe und $R_7$ ein Wasserstoffatom oder

A eine Gruppe der Formel

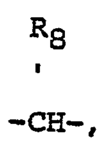

in welcher $R_8$ ein Wasserstoffatom, eine Hydroxy-, Alkanoyloxy- oder Alkoxycarbonyloxygruppe darstellt, und $R_7$ ein Wasserstoffatom oder $R_7$ und $R_8$ zusammen eine weitere Bindung darstellen,

E eine gegebenenfalls durch eine Alkylgruppe substituierte geradkettige Alkylengruppe mit 3 oder 4 Kohlenstoffatomen,

G eine gegebenenfalls durch eine Alkylgruppe substituierte geradkettige Alkylengruppe mit 2 bis 5 Kohlenstoffatomen,

$R_1$ ein Wasserstoff- oder Halogenatom, eine Trifluormethyl-, Nitro-, Amino-, Alkylamino-, Dialkylamino-, Alkyl-, Hydroxy-, Alkoxy- oder Phenylalkoxygruppe,

$R_2$ ein Wasserstoffatom oder Halogenatom, eine Hydroxy-, Alkoxy-, Phenylalkoxy- oder Alkylgruppe, oder

$R_1$ und $R_2$ zusammen eine Alkylendioxygruppe mit 1 oder 2 Kohlenstoffatomen,

$R_3$ ein Wasserstoffatom, eine Alkylgruppe oder eine Alkenylgruppe mit 3 bis 5 Kohlenstoffatomen,

$R_4$ ein Wasserstoff- oder Halogenatom, eine Alkyl-, Amino-, Alkylamino-, Dialkylamino-, Alkanoylamino-

, Alkoxycarbonylamino-, Alkylsulfonylamino-, Bis(alkylsulfonyl)-amino-, N-Alkyl-alkylsulfonylamino-, Cyano-, Alkylmercapto-, Alkylsulfinyl- oder Alkylsulfonylgruppe oder eine gegebenenfalls durch eine Alkyl-, Phenylalkyl-, 2-Hydroxyäthyl-, 3-Hydroxy-n-propyl-, 2-Hydroxy-n-propyl-, Alkylsulfonyl-, Cyanoalkyl-, Alkoxycarbonyl-, Hydroxycarbonylalkyl-, Alkoxycarbonylalkyl-, Trifluormethyl-, Difluormethyl- oder Trifluormethylsulfonylgruppe substituierte Hydroxygruppe,

$R_5$ ein Wasserstoff- oder Halogenatom, eine Alkyl-, Hydroxy-, Alkoxy-, Nitro-, Cyano- oder Trifluormethylgruppe oder

$R_4$ und $R_5$ zusammen eine Alkylendioxygruppe mit 1 oder 2 Kohlenstoffatomen und

$R_6$ ein Wasserstoff- oder Halogenatom, eine Alkyl- oder Alkoxygruppe, wobei alle vorstehend erwähnten Alkyl- oder Alkoxygruppen jeweils 1 bis 3 Kohlenstoffatome und die vorstehend erwähnten Alkanoylgruppen jeweils 2 oder 3 Kohlenstoffatome enthalten können.

Für die bei der Definition der Reste eingangs erwähnten Bedeutungen kommt beispielsweise

für $R_1$ die des Wasserstoff-, Fluor-, Chlor- oder Bromatoms, der Methyl-, Ethyl-, n-Propyl-, Isopropyl-, Trifluormethyl-, Hydroxy-, Methoxy-, Ethoxy-, n-Propoxy-, Isopropoxy-, Nitro-, Amino-, Methylamino-, Ethylamino-, n-Propylamino-, Isopropylamino-, Dimethylamino-, Diethylamino-, Di-n-propylamino-, Diisopropylamino-, Methyl-ethylamino-, Methyl-n-propylamino-, Methyl-isopropylamino-, Ethyl-n-propylamino-, Benzyloxy-, 1-Phenylethoxy-, 1-Phenylpropoxy-, 2-Phenylethoxy- oder 3-Phenylpropoxygruppe,

für $R_2$ die des Wasserstoff-, Chlor- oder Bromatoms, der Methyl-, Ethyl-, n-Propyl-, Isopropyl-, Hydroxy-, Methoxy-, Ethoxy-, n-Propoxy-, Isopropoxy-, Benzyloxy-, 1-Phenylethoxy-, 2-Phenylethoxy-, 2-Phenylpropoxy- oder 3-Phenylpropoxygruppe oder zusammen mit $R_1$ die der Methylendioxy- oder Ethylendioxygruppe,

für $R_3$ die des Wasserstoffatoms, der Methyl-, Ethyl-, n-Propyl-, Isopropyl-, Allyl-, Crotyl- oder n-Penten-2-yl-gruppe,

für $R_4$ die des Wasserstoff-, Fluor-, Chlor-, Brom- oder Jodatoms, der Methyl-, Ethyl-, n-Propyl-, Isopropyl-, Hydroxy-, Methoxy-, Ethoxy-, n-Propoxy-, Isopropoxy-, Amino-, Methylamino-, Ethylamino-, n-Propylamino-, Isopropylamino-, Dimethylamino-, Diethylamino-, Di-n-propylamino-, Diisopropylamino-, Methyl-ethylamino-, Methyl-n-propylamino-, Methyl-isopropylamino-, Ethyl-n-propylamino-, Acetylamino-, Propionylamino-, Methoxycarbonylamino-, Ethoxycarbonylamino-, n-Propoxycarbonylamino-, Methylsulfonylamino-, Ethylsulfonylamino-, n-Propylsulfonylamino-, Bis(methylsulfonyl)-amino-, Bis(ethylsulfonyl)-amino-, N-Methyl-methylsulfonylamino-, Cyano-, Methylmercapto-, Ethylmercapto-, n-Propylmercapto-, Methylsulfinyl-, Ethylsulfinyl-, Isopropylsulfinyl-, Methylsulfonyl-, Ethylsulfonyl-, n-Propylsulfonyl-, Benzyloxy-, 1-Phenylethoxy-, 2-Phenylethoxy-, 2-Phenyl-propoxy-, 3-Phenylpropoxy-, 2-Hydroxy-ethoxy-, 2-Hydroxy-n-propoxy-, 3-Hydroxy-n-propoxy-, Methylsulfonyloxy-, Ethylsulfonyloxy-, Isopropylsulfonyloxy-, Methoxycarbonyloxy-, Ethoxycarbonyloxy-, Isopropoxycarbonyloxy-, Hydroxycarbonylmethoxy-, 2-(Hydroxycarbonyl)-ethoxy-, Methoxycarbonylmethoxy-, Ethoxycarbonylmethoxy-, Isopropoxycarbonylmethoxy-, 2-(Methoxycarbonyl)-ethoxy-, 2-(n-Propoxycarbonyl)-ethoxy-, Cyanomethoxy-, 2-Cyanoethoxy-, 3-Cyano-n-propoxy-, Trifluormethoxy- oder Difluormethoxygruppe,

für $R_5$ die des Wasserstoff-, Chlor- oder Bromatoms, der Methyl-, Ethyl-, n-Propyl-, Isopropyl-, Hydroxy-, Methoxy-, Ethoxy-, n-Propoxy-, Isopropoxy-, Nitro-, Cyano- oder Trifluormethylgruppe oder $R_4$ und $R_5$ zusammen die der Methylendioxy- oder Ethylendioxygruppe,

für $R_6$ die des Wasserstoff-, Chlor- oder Bromatoms, der Methyl-, Ethyl-, n-Propyl-, Isopropyl-, Hydroxy-, Methoxy-, Ethoxy-, n-Propoxy- oder Isopropoxygruppe,

für $R_7$ die des Wasserstoffatoms oder zusammen mit $R_8$ die einer weiteren Bindung,

für A die der Methylen-, Hydroxymethylen-, Acetoxymethylen-, Propionyloxymethylen-, Methoxycarbonyloxymethylen-, Ethoxycarbonyl-oxymethylen-, n-Propoxycarbonyloxymethylen- oder Carbonylgruppe,

für E die der n-Propylen-, 1-Methyl-n-propylen, 2-Methyl-n-propylen-, 3-Methyl-n-propylen-, 1-Ethyl-n-propylen-, 2-n-Propyl-n-propylen-, 3-Ethyl-n-propylen-, n-Butylen-, 1-Methyl-n-butylen- oder 1-Ethyl-n-butylengruppe und

für G die der Ethylen-, 1-Methyl-ethylen-, 2-Ethyl-ethylen-, 1-Propyl-ethylen-, 2-Methyl-ethylen-, 2-Ethyl-ethylen-, n-Propylen-, n-Butylen-, n-Pentylen-, 1-Methyl-n-propylen-, 1-Methyl-n-butylen-, 1-Methyl-n-pentylen-, 1-Ethyl-n-propylen-, 2-Ethyl-n-propylen- oder 1-Ethyl-n-butylengruppe in Betracht.

Beispielsweise seien zusätzlich zu den in den Beispielen genannten Verbindungen noch folgende Verbindungen genannt, die unter die vorstehend erwähnte Formel I fallen:

6,7-Dimethoxy-2-[3-((2-(4-methylphenyl)-ethyl)-methylamino)-propyl]-1,2,3,4-tetrahydronaphthalin,

6,7-Dimethoxy-2-[3-((2-(3-methylphenyl)-ethyl)-methylamino)-propyl]-1,2,3,4-tetrahydronaphthalin,

6,7-Dimethoxy-2-[3-((2-(3-methoxyphenyl)-ethyl)-methylamino)-propyl]-1,2,3,4-tetrahydronaphthalin,

6,7-Dimethoxy-2-[3-((2-(4-difluormethoxyphenyl)-ethyl)-methylamino)-propyl]-1,2,3,4-tetrahydronaphthalin,

6,7-Dimethoxy-2-[3-((2-(4-(2-hydroxyethoxy)-phenyl)-ethyl)-methylamino)-propyl]-1,2,3,4-tetrahydronaphthalin,

6,7-Dimethoxy-2-[3-((3-(4-amino-3,5-dibrom-phenyl)-propyl)-methylamino)-propyl]-1,2,3,4-tetrahydronaphthalin,

6,7-Dimethoxy-2-[3-((2-(3,4-methylendioxyphenyl)-ethyl)-methylamino)-propyl]-1,2,3,4-tetrahydronaphthalin,

6,7-Dimethoxy-2-[3-((2-(3,4-dichlorphenyl)-ethyl)-methylamino)-propyl]-1,2,3,4-tetrahydronaphthalin,

6,7-Dimethoxy-2-[3-((3-(4-cyanophenyl)-propyl)-methylamino)-propyl]-1-hydroxy-1,2,3,4-tetrahydronaphthalin,

6,7-Dimethoxy-2-[3-((2-(4-methylsulfonylphenyl)-ethyl)-methylamino)-propyl]-1-hydroxy-1,2,3,4-tetrahydronaphthalin,

6,7-Dimethoxy-2-[3-((2-(4-methoxyphenyl)-ethyl)-methylamino)-propyl]-1-hydroxy-1,2,3,4-tetrahydronaphthalin,

6,7-Dimethoxy-2-[3-((2-phenyl-ethyl)-methylamino)-propyl]-1-hydroxy-1,2,3,4-tetrahydronaphthalin,

6,7-Dimethoxy-2-[3-((3-(4-bromphenyl)-propyl)-methylamino)-propyl]-1-hydroxy-1,2,3,4-tetrahydronaphthalin,

6,7-Dimethoxy-2-[3-((2-(4-methylphenyl)-ethyl)-methylamino)-propyl]-1-hydroxy-1,2,3,4-tetrahydronaphthalin,

6,7-Dimethoxy-2-[3-((2-(3-methylphenyl)-ethyl)-methylamino)-propyl]-1-hydroxy-1,2,3,4-tetrahydronaphthalin,

6,7-Dimethoxy-2-[3-((2-(3-methoxyphenyl)-ethyl)-methylamino)-propyl]-1-hydroxy-1,2,3,4-tetrahydronaphthalin,

6,7-Dimethoxy-2-[3-((2-(4-trifluormethylsulfonyloxyphenyl)-ethyl)-methylamino)-propyl]-1-hydroxy-1,2,3,4-tetrahydronaphthalin,

6,7-Dimethoxy-2-[3-((2-(4-cyanomethoxyphenyl)-ethyl)-methylamino)-propyl]-1-hydroxy-1,2,3,4-tetrahydronaphthalin,

6,7-Dimethoxy-2-[3-((2-(4-difluormethoxyphenyl)-ethyl)-methylamino)-propyl]-1-hydroxy-1,2,3,4-tetrahydronaphthalin,

6,7-Dimethoxy-2-[3-((2-(4-trifluormethoxyphenyl)-ethyl)-methylamino)-propyl]-1-hydroxy-1,2,3,4-tetrahydronaphthalin,

6,7-Dimethoxy-2-[3-((2-(4-methansulfonylaminophenyl)-ethyl)-methylamino)-propyl]-1-hydroxy-1,2,3,4-tetrahydronaphthalin,

6,7-Dimethoxy-2-[3-((2-(4-methoxycarbonylaminophenyl)-ethyl)-methylamino)-propyl]-1-hydroxy-1,2,3,4-tetrahydronaphthalin,

6,7-Dimethoxy-2-[3-((2-(4-ethoxycarbonylmethoxyphenyl)-ethyl)methylamino)-propyl]-1-hydroxy-1,2,3,4-tetrahydronaphthalin,

6,7-Dimethoxy-2-[3-((2-(4-hydroxycarbonylmethoxyphenyl)-ethyl)-methylamino)-propyl]-1-hydroxy-1,2,3,4-tetrahydronaphthalin,

6,7-Dimethoxy-2-[3-((3-(4-amino-3,5-dibrom-phenyl)-propyl)-methylamino)-propyl]-1-hydroxy-1,2,3,4-tetrahydronaphthalin,

6,7-Dimethoxy-2-[3-((2-(3,4-methylendioxyphenyl)-ethyl)-methylamino)-propyl]-1-hydroxy-1,2,3,4-tetrahydronaphthalin,

6,7-Dimethoxy-2-[3-((2-(3,4-dichlorphenyl)-ethyl)-methylamino)-propyl]-1-hydroxy-1,2,3,4-tetrahydronaphthalin,

6,7-Dimethoxy-2-[3-((3-(4-cyanophenyl)-propyl)-methylamino)-propyl]-1-oxo-1,2,3,4-tetrahydronaphthalin,

6,7-Dimethoxy-2-[3-((2-(4-methylsulfonylphenyl)-ethyl)-methyl-amino)-propyl]-1-oxo-1,2,3,4-tetrahydronaphthalin,

6,7-Dimethoxy-2-[3-((2-(4-methoxyphenyl)-ethyl)-methylamino)-propyl]-1-oxo-1,2,3,4-tetrahydronaphthalin,

6,7-Dimethoxy-2-[3-((2-phenyl-ethyl)-methylamino)-propyl]-1-oxo-1,2,3,4-tetrahydronaphthalin,

6,7-Dimethoxy-2-[3-((3-(4-bromphenyl)-propyl)-methylamino)-propyl]-1-oxo-1,2,3,4-tetrahydronaphthalin,

6,7-Dimethoxy-2-[3-((2-(4-methylphenyl)-ethyl)-methylamino)-propyl]-1-oxo-1,2,3,4-tetrahydronaphthalin,

6,7-Dimethoxy-2-[3-((2-(3-methylphenyl)-ethyl)-methylamino)-propyl]-1-oxo-1,2,3,4-tetrahydronaphthalin,

6,7-Dimethoxy-2-[3-((2-(3-methoxyphenyl)-ethyl)-methylamino)-propyl]-1-oxo-1,2,3,4-tetrahydronaphthalin,

6,7-Dimethoxy-2-[3-((2-(4-trifluormethylsulfonyloxyphenyl)-ethyl)-methylamino)-propyl]-1-oxo-1,2,3,4-tetrahydronaphthalin,

6,7-Dimethoxy-2-[3-((2-(4-cyanomethoxyphenyl)-ethyl)-methylamino)-propyl]-1-oxo-1,2,3,4-tetrahydronaphthalin,

6,7-Dimethoxy-2-[3-((2-(4-difluormethoxyphenyl)-ethyl)-methyl-amino)-propyl]-1-oxo-1,2,3,4-tetrahydronaphthalin,

6,7-Dimethoxy-2-[3-((2-(4-trifluormethoxyphenyl)-ethyl)-methylamino)-propyl]-1-oxo-1,2,3,4-tetrahydronaphthalin,

6,7-Dimethoxy-2-[3-((2-(4-methansulfonylaminophenyl)-ethyl)-methylamino)-propyl]-1-oxo-1,2,3,4-tetrahydronaphthalin,

6,7-Dimethoxy-2-[3-((2-(4-methoxycarbonylaminophenyl)-ethyl)-methylamino)-propyl]-1-oxo-1,2,3,4-tetrahydronaphthalin,

6,7-Dimethoxy-2-[3-((2-(4-ethoxycarbonylmethoxyphenyl)-ethyl)-methylamino)-propyl]-1-oxo-1,2,3,4-tetrahydronaphthalin,

6,7-Dimethoxy-2-[3-((2-(4-hydroxycarbonylmethoxyphenyl)-ethyl)-methylamino)-propyl]-1-oxo-1,2,3,4-tetrahydronaphthalin,

6,7-Dimethoxy-2-[3-((3-(4-amino-3,5-dibrom-phenyl)-propyl)-methylamino)-propyl]-1-oxo-1,2,3,4-tetrahydronaphthalin,

6,7-Dimethoxy-2-[3-((2-(3,4-methylendioxyphenyl)-ethyl)-methylamino)-propyl]-1-oxo-1,2,3,4-tetrahydronaphthalin und

6,7-Dimethoxy-2-[3-((2-(3,4-dichlorphenyl)-ethyl)-methylamino)-propyl]-1-oxo-1,2,3,4-tetrahydronaphthalin, deren Enantiomere, deren Diastereomere und deren Säureadditionssalze.

Bevorzugte Verbindungen der obigen Formel I sind jedoch diejenigen, in denen

n die Zahl 1 oder 2,

A eine Carbonylgruppe und $R_7$ ein Wasserstoffatom oder

A eine Gruppe der Formel

$$\begin{array}{c} R_8 \\ | \\ -CH-, \end{array}$$

in welcher $R_8$ ein Wasserstoffatom oder eine Hydroxygruppe bedeutet, und $R_7$ ein Wasserstoffatom oder $R_7$ und $R_8$ zusammen eine weitere Bindung darstellen,

E die n-Propylengruppe,

G die Ethylen- oder n-Propylengruppe,

$R_1$ eine Methyl- oder Methoxygruppe,

$R_2$ ein Wasserstoffatom oder eine Methoxygruppe,

$R_3$ die Methylgruppe,

$R_4$ ein Wasserstoff-, Chlor- oder Bromatom, eine Hydroxy-, Methoxy-, Cyano-, Cyanomethoxy-, Hydroxycarbonylmethoxy-, Methoxycarbonylmethoxy-, Ethoxycarbonylmethoxy-, Methyl-, Amino-, Acetylamino-, Methoxycarbonylamino-, Methyl sulfonyloxy-, Trifluormethylsulfonyloxy-, Benzyloxy-, Methylsulfonylamino- oder Bis(methylsulfonyl)aminogruppe,

$R_5$ ein Wasserstoff-, Chlor- oder Bromatom, eine Methoxy- oder Nitrogruppe und

$R_6$ ein Wasserstoff-, Chlor- oder Bromatom bedeuten, deren Enantiomere, deren Diastereomere und deren Säureadditionssalze.

Besonders bevorzugte Verbindungen der Formel I sind diejenigen, in denen

$R_1$ bis $R_3$, A, E, G und n wie vorstehend definiert sind,

$R_4$ eine Methoxygruppe,

$R_5$ eine Methoxygruppe und

$R_6$ ein Wasserstoffatom bedeuten, deren Enantiomere, deren Diastereomere und deren Säureadditionssalze.

Erfindungsgemäß erhält man die neuen Verbindungen nach folgenden Verfahren:

a) Zur Herstellung von Verbindungen der Formel I, in der $R_7$ und $R_8$ je ein Wasserstoffatom oder zusammen eine weitere Bindung darstellen:

Umsetzung einer Verbindung der Formel

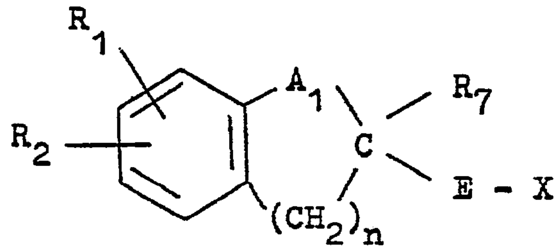

(II)

in der
$R_1$, $R_2$, n und E wie eingangs definiert sind,

$A_1$ eine Gruppe der Formel

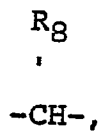

in welcher $R_8$ ein Wasserstoffatom bedeutet, und $R_7$ ein Wasserstoffatom oder $R_7$ und $R_8$ zusammen eine weitere Bindung darstellen, und

X eine nukleophile Austrittsgruppe wie ein Halogenatom oder eine Sulfonyloxygruppe darstellt, z.B. ein Chlor-, Brom- oder Jodatom, die Methansulfonyloxy-, p-Toluolsulfonyloxy- oder Ethoxysulfonyloxygruppe, mit einem Amin der Formel

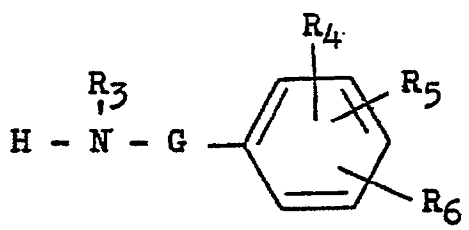

(III)

in der
$R_3$ bis $R_6$ und G wie eingangs definiert sind.

Die Umsetzung wird zweckmäßigerweise in einem Lösungsmittel oder Lösungsmittelgemisch wie Aceton, Diethylether, Methylformamid, Dimethylformamid, Dimethylsulfoxid, Benzol, Chlorbenzol, Tetrahydrofuran, Benzol/Tetrahydrofuran, Dioxan oder in einem Überschuß der eingesetzten Verbindungen der Formeln II und/oder III und gegebenenfalls in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid, eines Alkalihydrids wie Natriumhydrid, einer tertiären organischen Base wie Triethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes zweckmäßigerweise bei Temperaturen zwischen 0 und 150°C, vorzugsweise bei Temperaturen zwischen 50 und 120°C, z.B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base oder eines Überschusses des eingesetzten Amins der allgemeinen Formel III durchgeführt.

b) Umsetzung einer Verbindung der Formel

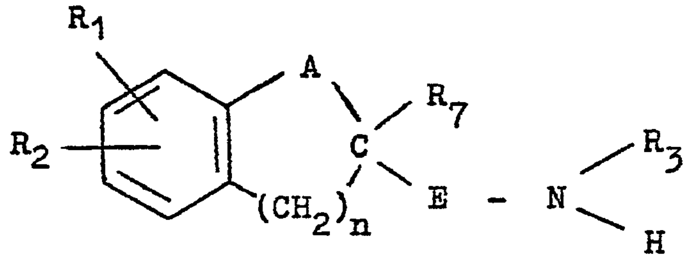

(IV)

in der
n, A, E, $R_1$ bis $R_3$ und $R_7$ wie eingangs definiert sind,
mit einer Verbindung der Formel

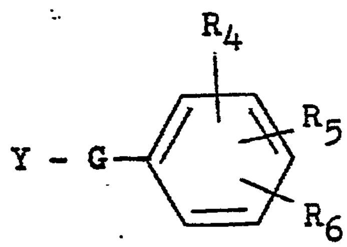

(V)

in der
$R_4$ bis $R_6$ und G wie eingangs definiert sind und
Y eine nukleophile Austrittsgruppe wie ein Halogenatom oder eine Sulfonyloxygruppe darstellt, z.B. ein Chlor-, Brom- oder Jodatom, die Methansulfonyloxy-, p-Toluolsulfonyloxy- oder Ethoxysulfonyloxygruppe.

Die Umsetzung wird zweckmäßigerweise in einem Lösungsmittel oder Lösungsmittelgemisch wie Aceton, Diethylether, Methylformamid, Dimethylformamid, Dimethylsulfoxid, Benzol, Chlorbenzol, Tetrahydrofuran, Benzol/Tetrahydrofuran, Dioxan oder in einem Überschuß der eingesetzten Verbindungen der Formeln IV und/oder V und gegebenenfalls in Gegenwart eines säurebindenden Mittels, z.B. eines Alkoholats wie Kalium-tert.butylat, eines Alkalihydroxids wie Natrium- oder Kaliumhydroxid, eines Alkalicarbonats wie Kaliumcarbonat, eines Alkaliamids wie Natriumamid, eines Alkalihydrids wie Natriumhydrid, einer tertiären organischen Base wie Triethylamin oder Pyridin, wobei die letzteren gleichzeitig auch als Lösungsmittel dienen können, oder eines Reaktionsbeschleunigers wie Kaliumjodid je nach der Reaktionsfähigkeit des nukleophil austauschbaren Restes zweckmäßigerweise bei Temperaturen zwischen 0 und 150°C, vorzugsweise bei Temperaturen zwischen 50 und 120°C, z.B. bei der Siedetemperatur des verwendeten Lösungsmittels, durchgeführt. Die Umsetzung kann jedoch auch ohne Lösungsmittel durchgeführt werden. Besonders vorteilhaft wird die Umsetzung jedoch in Gegenwart einer tertiären organischen Base oder eines Überschusses des eingesetzten Amins der Formel IV durchgeführt.

c) Zur Herstellung von Verbindungen der Formel I, in der A eine Gruppe der Formel

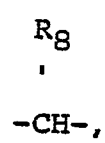

darstellt, in welcher $R_8$ ein Wasserstoffatom oder eine Hydroxygruppe bedeutet:

Reduktion einer Verbindung der Formel

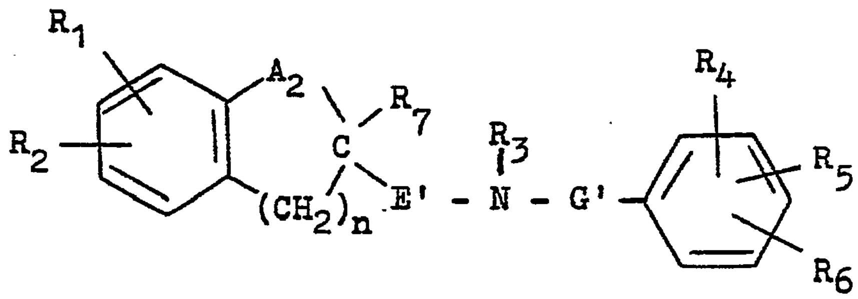

(VI)

in der
n und $R_1$ bis $R_6$ wie eingangs definiert sind,

$A_2$ eine Carbonylgruppe und $R_7$ ein Wasserstoffatom oder
$A_2$ eine Gruppe der Formel

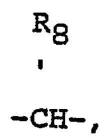

in welcher $R_8$ ein Wasserstoffatom oder eine Hydroxygruppe darstellt, und $R_7$ ein Wasserstoffatom,

E′ die für E oder G′ die für G eingangs erwähnten Bedeutungen besitzt, wobei jedoch in den Resten E oder G eine zu der

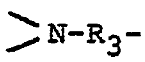

-Gruppe benachbarte Methylengruppe durch eine Carbonylgruppe ersetzt sein muß.

Die Reduktion wird vorzugsweise mit einem Metallhydrid wie Lithiumaluminiumhydrid oder Diboran oder mit einem Komplex aus Boran und einem Thioäther, z.B. mit Boran-Dimethylsulfid-Komplex, gegebenenfalls in Gegenwart einer Lewis-Säure wie Bortrifluorid in einem geeigneten Lösungsmittel wie Diäthyläther oder Tetrahydrofuran bei Temperaturen zwischen 0 und 80°C, vorzugsweise jedoch bei der Siedetemperatur des verwendeten Lösungsmittels, z.B. bei Temperaturen zwischen 35 und 65°C, durchgeführt.

Bedeutet $A_2$ in einer eingesetzten Verbindung der Formel VI eine Carbonylgruppe oder $R_8$ eine Hydroxygruppe, so wird zur Herstellung von Verbindungen der Formel I, in der $R_8$ ein Wasserstoffatom darstellt, die Reduktion vorzugsweise in Gegenwart einer Lewis-Säure, z.B. mit Boran-Dimethylsulfid-Komplex in Gegenwart von Bortrifluorid, oder

bedeutet $A_2$ in einer eingesetzten Verbindung der Formel VI die Carbonylgruppe oder $R_8$ eine Hydroxygruppe, so wird zur Herstellung von Verbindungen der allgemeinen Formel I, in der $R_8$ eine Hydroxygruppe darstellt, die Reduktion vorzugsweise mit Lithiumaluminiumhydrid durchgeführt.

d) Zur Herstellung von Verbindungen der Formel I, in der A die Carbonylgruppe darstellt:

Oxidation einer Verbindung der Formel

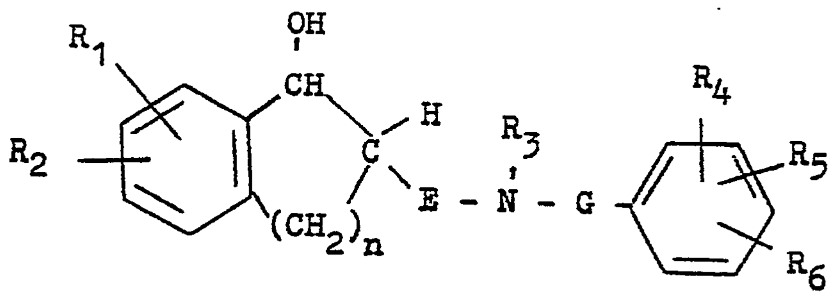

(VII)

in der
n, E, G und $R_1$ bis $R_6$ wie eingangs definiert sind.

Die Oxidation wird vorzugsweise mit einem Oxidationsmittel wie Kaliumpermanganat, Bariummanganat, Kaliumdichromat oder mit einem Keton in Gegenwart einer Base (Oppenauer-Methode), z.B. mit Aceton/Aluminiumisopropylat oder Benzophenon/Kalium-tert.butylat, in einem geeigneten Lösungsmittel wie Wasser, Wasser/Dioxan, Eisessig, Wasser/Essigsäure oder Toluol bei Temperaturen zwischen 0 und 150°C durchgeführt. Die Oxidation mit einem anorganischen Oxidationsmittel wird jedoch vorzugsweise bei Temperaturen zwischen 0 und 50°C und die Oxidation nach Oppenauer vorzugsweise bei der Siedetemperatur des Reaktionsgemisches, z.B. bei Temperaturen zwischen 50 und 115°C, durchgeführt.

e) Zur Herstellung von Verbindungen der Formel I, in der $R_4$ eine Alkylamino-, Dialkylamino-, Alkanoylamino-, Alkoxycarbonylamino-, Alkylsulfonylamino-, Bis(alkylsulfonyl)-amino-, N-Alkyl-alkylsulfonylamino-, Alkylmercapto-, Alkoxy-, Alkoxycarbonyloxy-, Hydroxycarbonylalkoxy-, Alkoxycarbonylalkoxy-, Phenylalkoxy-, Trifluormethoxy-, Difluormethoxy-, Cyanoalkoxy-, Alkylsulfonyloxy- oder Trifluormethylsulfonyloxygruppe darstellt:

Umsetzung einer Verbindung der Formel

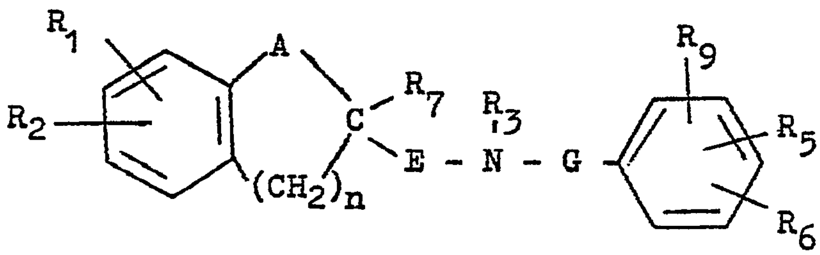

(VIII)

in der
$R_1$ bis $R_3$, $R_5$ bis $R_7$, A, E, G und n wie eingangs definiert sind und
$R_9$ eine Hydroxy-, Amino- oder Alkylaminogruppe mit 1 bis 3 Kohlenstoffatomen darstellt, mit einer Verbindung der Formel

Z - $R_{10}$(IX)

in der
Z eine nukleophile Austrittsgruppe wie ein Halogenatom, z.B. ein Chlor-, Brom- oder Jodatom, und
$R_{10}$ eine Alkyl-, Alkanoyl-, Alkoxycarbonyl-, Hydroxycarbonylalkyl-, Alkoxycarbonylalkyl-, Alkylsulfonyl-, Phenylalkyl-, Trifluormethyl-, Difluormethyl- oder Cyanoalkylgruppe bedeuten, wobei die vorstehend erwähnten Alkyl- und Alkoxyteile jeweils 1 bis 3 Kohlenstoffatome bzw. der Alkanoylteil 2 oder 3 Kohlenstoffatome enthalten kann.

Die Umsetzung wird zweckmäßigerweise in einem Lösungsmittel oder Lösungsmittelgemisch wie Methylenchlorid, Chloroform, Tetrachlorkohlenstoff, Ether, Tetrahydrofuran, Dioxan, Benzol, Toluol, Acetonitril oder Dimethylformamid, gegebenenfalls auch in Gegenwart eines die Säure aktivierenden Mittels oder eines wasserentziehenden Mittels, z. B. in Gegenwart von Chlorameisensäureethylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol, und gegebenenfalls in Gegenwart einer anorganischen Base wie Natriumcarbonat oder einer tertiären organischen Base wie Triethylamin oder Pyridin, wobei die beiden letzteren auch gleichzeitig als Lösungsmittel dienen können, bei Temperaturen zwischen -25 und 250°C, vorzugsweise jedoch bei Temperaturen zwischen -10°C und der Siedetemperatur des verwendeten Lösungsmittels durchgeführt werden.

g) Zur Herstellung von Verbindungen der Formel I, in der $R_7$ und $R_8$ zusammen eine weitere Bindung darstellen:

Dehydratisierung einer Verbindung der Formel

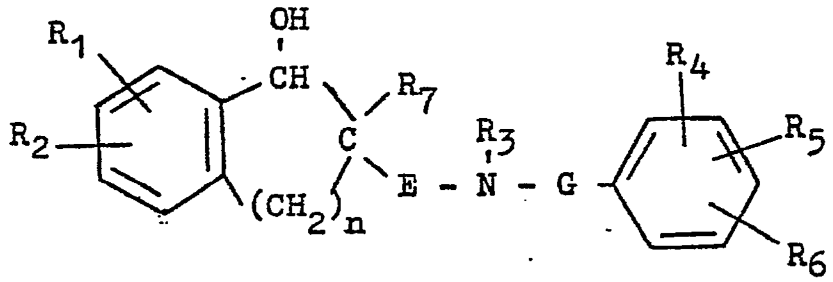

(X)

in der
n, E, G und $R_1$ bis $R_7$ wie eingangs definiert sind.

Die Dehydratisierung wird zweckmäßigerweise in einem Lösungsmittel wie Aceton, Methanol, Ethanol, Tetrahydrofuran oder Dioxan und vorzugsweise in Gegenwart einer Säure wie Salzsäure oder Schwefelsäure, vorzugsweise in Gegenwart einer alkoholischen Säure wie methanolischer Salzsäure, bei Temperaturen zwischen 0 und 50°C, vorzugsweise bei Raumtemperatur, durchgeführt.

Bei den vorstehend beschriebenen Umsetzungen können gegebenenfalls vorhandene reaktive Gruppen wie Hydroxy-, Amino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Benzoyl- oder Tetrahydropyranylgruppe und
als Schutzrest für eine Imino- oder Aminogruppe die Acetyl-, Benzoyl-, Ethoxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

Erhält man erfindungsgemäß eine Verbindung der Formel I, in der $R_4$ eine Benzyloxygruppe darstellt, so kann diese mittels Entbenzylierung in die entsprechende Hydroxyverbindung übergeführt werden.

Die nachträgliche Entbenzylierung wird vorzugsweise in einem Lösungsmittel wie Wasser, Wasser/Ethanol, Methanol, Eisessig, Essigsäureethylester oder Dimethylformamid zweckmäßi gerweise mit Wasserstoff in Gegenwart eines Hydrierungskatalysators wie Raney-Nickel, Platin oder Palladium/Kohle bei Temperaturen zwischen 0 und 50°C, vorzugsweise jedoch bei Raumtemperatur, durchgeführt.

Die erhaltenen Verbindungen der Formel I lassen sich, sofern diese mindestens ein chirales Zentrum besitzen, mittels üblichen Methoden in ihre Diastereomeren, beispielsweise durch Säulenchromatographie, und in ihre Enantiomeren auftrennen, beispielsweise durch Säulenchromatographie an einer chiralen Phase oder durch Kristallisation mit optisch aktiven Säuren, z.B. mit D- oder L-Monomethylweinsäure, D- oder L-Diacetylweinsäure, D- oder L-Weinsäure, D- oder L-Milchsäure, D- oder L-Camphersäure, D- oder L-Dibenzoylweinsäure, D- oder L-Camphersulfonsäure oder D- oder L-Camphansäure.

Die erhaltenen Verbindungen der Formel I lassen sich ferner in ihre Säureadditionssalze, insbesondere für ihre pharmazeutische Anwendung in ihre physiologisch verträglichen Säureadditionssalze mit anorganischen oder organischen Säuren überführen. Als Säuren kommen hierbei beispielsweise Salzsäure, Bromwasserstoffsäure, Schwefelsäure, Phosphorsäure, Essigsäure, Milchsäure, Zitronensäure, Weinsäure, Bernsteinsäure, Maleinsäure, Fumarsäure oder Oxalsäure in Betracht.

Die als Ausgangsstoffe verwendeten Verbindungen der Formeln II bis X sind teilweise literaturbekannt bzw. man erhält sie nach an sich bekannten Verfahren.

Beispielsweise erhält man eine Ausgangsverbindung der allgemeinen Formel II durch Reduktion einer entsprechenden Carbonsäure der Formel

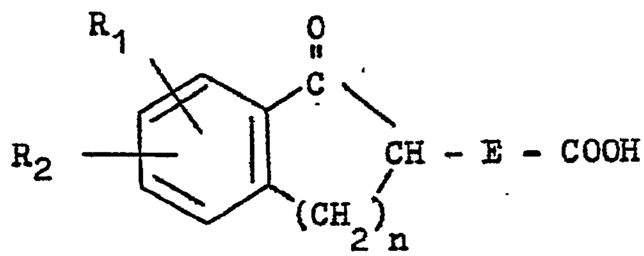

(XI)

in der

$R_1$, $R_2$, E und n wie eingangs definiert sind, oder eines entsprechenden Esters zum entsprechenden Alkohol und anschließende Umsetzung mit einem Halogenierungsmittel wie Phosphortribromid oder Bromwasserstoff. Die hierfür benötigte Carbonsäure der allgemeinen Formel XI erhält man durch Michael-Addition eines entsprechenden Acrylesters an eine Verbindung der Formel

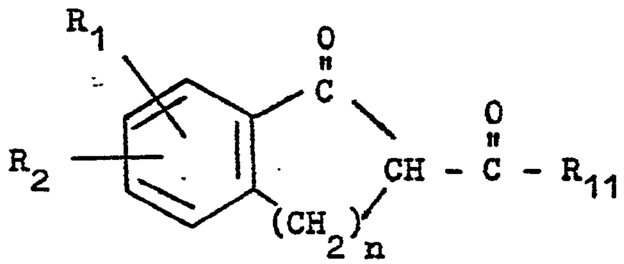

(XII)

in der

$R_1$, $R_2$ und n wie eingangs definiert sind und

$R_{11}$ ein Wasserstoffatom oder eine niedere Alkoxygruppe darstellt. Die hierfür erforderliche Verbindung der Formel XII erhält man wiederum durch Umsetzung eines entsprechenden Ketons mit einem Ameisensäureester oder einem Dialkylcarbonat in Gegenwart einer starken Base wie einem Alkalialkoholat oder einem Alkalihydrid.

Eine Ausgangsverbindung der Formel IV erhält man beispielsweise durch Umsetzung eines Carbonsäure der Formel XI mit einem entsprechenden Amin in Gegenwart von N,N'-Carbonyl-diimidazol und anschließende Reduktion des so erhaltenen Amids.

Eine als Ausgangsstoff verwendete Verbindung der Formeln VI, VII, VIII und X erhält man durch Umsetzung einer entsprechenden ω-Halogenverbindung mit einem entsprechenden Amin.

Wie bereits eingangs erwähnt, weisen die neuen Verbindungen der Formel I und deren physiologisch verträgliche Säureadditionssalze mit anorganischen oder organischen Säuren wertvolle pharmakologische Eigenschaften auf, insbesondere bei geringen zentralen Nebenwirkungen eine besonders herzfrequenzsenkende Wirkung sowie eine Herabsetzung des $O_2$-Bedarfs des Herzens.

Beispielsweise wurden die Verbindungen

A = 6,7-Dimethoxy-2-[3-((2-(3,4-dimethoxyphenyl)-ethyl)-methylamino)-propyl]-1-oxo-1,2,3,4-tetrahydro-naphthalin,

B = 6,7-Dimethoxy-2-[3-((2-(3,4-dimethoxyphenyl)-ethyl)-methylamino)-propyl]-1,2,3,4-tetrahydronaphthalin-hydrochlorid und

C = 5,6-Dimethoxy-2-[3-((2-(3,4-dimethoxyphenyl)-ethyl)-methylamino)-propyl]-1-hydroxy-indan

auf ihre biologischen Eigenschaften wie folgt untersucht:

Wirkung auf die Herzfrequenz an Ratten:

Die Wirkung der zu untersuchenden Substanzen auf die Herzfrequenz wurde pro Dosis an 2 Ratten mit einem durchschnittlichen Gewicht von 250-300 g untersucht. Hierzu wurden die Ratten mit Pentobarbital (50 mg/kg i.p. und 10 mg/kg s.c.) narkotisiert. Die zu untersuchenden Substanzen wurden in wäßriger Lösung in die Vena jugularis injiziert (0,1 ml/100 g).

Der Blutdruck wurde über in eine A. carotis eingebundene Kanüle gemessen und die Herzfrequenz wurde aus einem mit Nadelelektroden abgeleiteten EKG (II. oder III. Ableitung) registriert. Die Herzfrequenz der Tiere in der Kontrollperiode lagen zwischen 350 und 400 Schlägen/Minuten (S/min).

Die nachfolgende Tabelle enthält die gefundenen Werte:

| Substanz | Dosis [mg/kg] | Herzfrequenzsenkung, gemessen 5 Minuten nach Substanzapplikation [S/min] |
|---|---|---|
| A | 5,0 | 157 |
| B | 5,0 | 150 |
| C | 5,0 | 152 |

Die erfindungsgemäß hergestellten Verbindungen weisen in therapeutischen Dosen keinerlei toxische Nebenwirkungen auf. So konnten beipielsweise bei einer intravenösen Applikation der Substanz A und C auch in einer hohen Dosis von 10 mg/kg an Mäusen, außer einer geringen Sedation keine toxischen Nebenwirkungen beobachtet werden.

Aufgrund ihrer pharmakologischen Eigenschaften eignen sich die erfindungsgemäß hergestellen Verbindungen zur Behandlung von Sinustachykardien verschiedener Genese und zur Prophylaxe und Therapie ischämischer Herzerkrankungen.

Die zur Erzielung einer entsprechenden Wirkung erforderlichen Dosierung beträgt zweckmäßigerweise ein- bis zweimal täglich 0,03 bis 0,4 mg/kg Körpergewicht, vorzugsweise 0,07 bis 0,25 mg/kg Körpergewicht. Hierzu lassen sich die erfindungsgemäß hergestellten Verbindungen der Formel I sowie ihre physiologlisch verträglichen Säureadditionssalze mit anorganischen oder organischen Säuren, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Carboxymethylcellulose oder fetthaltige Substanzen wie Hartfett oder deren geeignete Gemische, in übliche galenische Zubereitungen wie Tabletten, Dragees, Kapseln, Pulver, Suspensionen, Tropfen, Ampullen, Säfte oder Zäpfchen einarbeiten.

Die nachfolgenden Beispiele sollen die Erfindung näher erläutern:

Beispiel A

6,7-Dimethoxy-1-oxo-1,2,3,4-tetrahydronaphthalin-2-carbaldehyd

187,5 g (0,909 Mol) 6,7-Dimethoxy-1-oxo-1,2,3,4-tetrahydronaphthalin werden in 4,0 1 Diethylether suspendiert und unter Rühren mit 123,4 g (1,1 Mol) Kalium-tert.butylat versetzt. Nach 30 Minuten werden zu dem entstandenen Niederschlag 89 ml (1,1 Mol) Ameisensäureethylester zugetropft. Nach 5 Stunden wird die blaurote Suspension mit 1 1 Wasser versetzt Die Wasserphase wird abgetrennt und mit konzentrierter Salzsäure angesäuert, wobei ein gelber Niederschlag ausfällt. Die salzsaure Phase wird 3 x mit Methylenchlorid extrahiert, über Magnesiumsulfat getrocknet und im Vakuum eingedampft.
Ausbeute 186,2 g (87,4 % der Theorie),
Schmelzpunkt: 153-154°C.

Beispiel B

3-(6,7-Dimethoxy-1-oxo-1,2,3,4-tetrahydronaphthalin-2-yl)-propionsäure-methylester

Ein Gemisch von 128,8 g (0,55 Mol) 6,7-Dimethoxy-1-oxo-1,2,3,4-tetrahydronaphthalin-2-carbaldehyd, 125 ml (1,38 Mol) Acrylsäuremethylester und 82,5 ml (0,6 Mol) Triethylamin wird 6 Stunden unter Rückfluß erhitzt. Anschließend wird im Vakuum eingedampft und der erhaltene Rückstand über 1600 g Aluminiumoxid (neutral, Aktivität II-III) mit Methylenchlorid und anschließend mit steigenden Anteilen von Ethanol (bis 1 %) chromatographiert.
Ausbeute: 144,5 g (90 % der Theorie),
Schmelzpunkt: 104-106°C.

Beispiel C

3-(6,7-Dimethoxy-1-oxo-1,2,3,4-tetrahydronaphthalin-2-yl)-propionsäure

169 g (0,578 Mol) 3-(6,7-Dimethoxy-1,2,3,4-tetrahydronaphthalin-2-yl)-propionsäuremethylester werden in 1,5 1 8%iger Natronlauge suspendiert und 90 Minuten unter Rückfluß erhitzt. Danach wird auf Eis

gegossen und mit konzentrierter Salzsäure angesäuert. Der entstandene Niederschlag wird abgesaugt.
Ausbeute: 152,1 g (94,5 % der Theorie),
Schmelzpunkt: 156-158°C.

Beispiel D

3-(6,7-Dimethoxy-1-oxo-1,2,3,4-tetrahydronaphthalin-2-yl)-N-(2-(3,4-dimethoxyphenyl)-ethyl)-N-methyl-propionsäureamid

8,35 g (0,03 Mol) 3-(6,7-Dimethoxy-1-oxo-1,2,3,4-tetrahydronaphthalin-2-yl)-propionsäureamid werden in 120 ml Essigester suspendiert, mit 4,86 g (0,03 Mol) N,N'-Carbonyldiimidazol versetzt und 60 Minuten gerührt. Zu dieser Suspension werden 5,86 g (0,030 Mol) N-Methyl-homoveratrylamin, gelöst in 30 ml Essigester, gegeben. Nach einer Stunde wird 2 × mit 8%iger Natronlauge extrahiert. Die organische Phase wird über Magnesiumsulfat getrocknet und im Vakuum eingedampft.
Ausbeute: 12,4 g (91 % der Theorie),
$R_f$-Wert: 0,8 (Aluminiumoxid, neutral; Laufmittel: 3 % Ethanol in Methylenchlorid).

Beispiel E

3-(6,7-Dimethoxy-1-oxo-1,2,3,4-tetrahydronaphthalin-2-yl)-N-(2-(4-benzyloxyphenyl)-ethyl)-N-methyl-propionsäureamid

Hergestellt analog Beispiel D aus 3-(6,7-Dimethoxy-1-oxo-1,2,3,4-tetrahydronaphthalin-2-yl)-propionsäure, N,N'-Carbonyldiimidazol und N-Methyl-(2-(4-benzyloxyphenyl)-ethylamin.
Ausbeute: 91 % der Theorie,
Schmelzpunkt: 126-128°C.

Beispiel F

3-(6,7-Dimethoxy-1-oxo-1,2,3,4-tetrahydronaphthalin-2-yl)-N-(2-(4-nitrophenyl)-ethyl)-N-methyl-propionsäureamid

Hergestellt analog Beispiel D aus 3-(6,7-Dimethoxy-1-oxo-1,2,3,4-tetrahydronaphthalin-2-yl)-propionsäure, N,N'-Carbonyldiimidazol und N-Methyl-2-(4-nitrophenyl)ethylamin.
Ausbeute: 87,8 % der Theorie,
Schmelzpunkt: 128-130°C.

Beispiel G

3-(6,7-Dimethoxy-1-oxo-1,2,3,4-tetrahydronaphthalin-2-yl)-N-(2-(4-aminophenyl)-ethyl)-N-methyl-propionsäureamid.

10,0 g (0,023 Mol) 3-(6,7-Dimethoxy-1-oxo-1,2,3,4-tetrahydronaphthalin-2-yl)-N-(2-(4-nitrophenyl)-ethyl)-N-methyl)propionsäureamid werden in 120 ml Methanol in Anwesenheit von 1,0 g 10%iger Palladium/Kohle 2 Stunden bei Raumtemperatur und 5 bar Wasserstoff hydriert. Anschließend wird der Katalysator abgesaugt und das Methanol im Vakuum abdestilliert.
Ausbeute: 9,3 g (100 % der Theorie),
$R_f$-Wert: 0,4 (Aluminiumoxid, neutral; Laufmittel: 3 % Ethanol in Methylenchlorid).

Beispiel H

5,6-Dimethoxy-1-oxo-indan-2-carbaldehyd

Hergestellt analog Beispiel A aus 5,6-Dimethoxy-1-oxo-indan.
Ausbeute: 81 % der Theorie,
Schmelzpunkt: 145-147°C.

Beispiel I

3-(5,6-Dimethoxy-1-oxo-indan-2-yl)-propionsäure-methylester

Hergestellt aus 5,6-Dimethoxy-1-oxo-indan-2-carbaldehyd und Acrylsäure-methylester analog Beispiel B.
Ausbeute 38 % der Theorie,
Schmelzpunkt: 59-62°C.

Beispiel K

3-(5,6-Dimethoxy-1-oxo-indan-2-yl)-propionsäure

Hergestellt aus 3-(5,6-Dimethoxy-1-oxo-indan-2-yl)-propionsäure-methylester analog Beispiel C.
Ausbeute: 60 % der Theorie,
Schmelzpunkt: 146-148°C.

Beispiel L

3-(5,6-Dimethoxy-1-oxo-indan-2-yl)-N-((2-(3,4-dimethoxy-phenyl)-ethyl)-N-methylpropionsäureamid

Hergestellt aus 3-(5,6-Dimethoxy-1-oxo-indan-2-yl)-propionsäure und N-Methylhomoveratrylamin analog Beispiel D.
Ausbeute: 83 % der Theorie,
$R_f$-Wert: 0,48 (Aluminiumoxid N; Methylenchlorid + 2 % Ethanol)

Beispiel M

6,7-Dimethoxy-2-(3-methylaminopropyl)-1,2,3,4-tetrahydronaphthalin

a) 3-(6,7-Dimethoxy-1-oxo-1,2,3,4-tetrahydronaphthalin-2-yl)-N-methylpropionsäureamid

8,35 g (0,03 Mol) 3-(6,7-Dimethoxy-1-oxo-1,2,3,4-tetrahydronaphthalin-2-yl)-propionsäure werden in 120 ml Essigester suspendiert, mit 4,86 g (0,03 Mol) N,N'-Carbonyldiimidazol versetzt und 30 Minuten bei 50°C gerührt. Zu dieser Suspension werden innerhalb 30 Minuten 4,65 g (0,15 Mol) Methylamin eingeleitet. Danach wird nochmals 30 Minuten nachgerührt, der Niederschlag abgesaugt und mit kaltem Essigester gewaschen.
Ausbeute: 6,9 g (79 % der Theorie),
Schmelzpunkt: 160-161°C,
Ber.: C 65,96 H 7,27 N 4,81
Gef.: C 66,15 H 7,29 N 4,96

b) 6,7-Dimethoxy-2-(3-methylaminopropyl)-1,2,3,4-tetrahydronaphthalin

6,7 g (0,023 Mol) 3-(6,7-Dimethoxy-1-oxo-1,2,3,4-tetrahydronaphthalin-2-yl)-N-methyl-propionsäureamid werden in 80 ml Tetrahydrofuran suspendiert. Dazu werden unter Stickstoff 3,26 g (0,023 Mol) Bortrifluorid-etherat gegeben und alles auf 60°C erwärmt. Bei dieser Temperatur werden nun 5,8 ml (0,058 Mol) Borandimethylsulfidkomplex (10 molare Lösung) zugetropft. Anschließend wird 5 Stunden unter Rückfluß erhitzt. Das abgekühlte Reaktionsgemisch wird tropfenweise mit Methanol versetzt, dann werden 10 ml methanolische Salzsäure zugegeben und nochmals 2 Stunden unter Rückfluß erhitzt. Der Ansatz wird nach Abdampfen des Lösungsmittels in 2 molarer Natronlauge aufgenommen und mit Essigester ausgeschüttelt. Die organischen Phasen werden über Magnesiumsulfat getrocknet im Vakuum eingedampft und der Rückstand über Aluminiumoxid (neutral, Aktivität II-III) mit Methylenchlorid und ansteigenden Teilen methanolischen Ammoniaks gereinigt.
Ausbeute: 33 % der Theorie,
$R_f$-Wert: 0,15 (Aluminiumoxid neutral, Laufmittel: 3 % Ethanol in Methylenchlorid)

Beispiel N

6,7-Dimethoxy-2-(3-brompropyl-1)-1,2,3,4-tetrahydronaphthalin

a) 3-(6,7-Dimethoxy-1,2,3,4-tetrahydronaphthalin-2-yl)-propanol-1

Hergestellt analog Beispiel Mb aus 3-(6,7-Dimethoxy-1-oxo-1,2,3,4-tetrahydronaphthalin-2-yl)-propionsäure.
Ausbeute: 84 % der Theorie,
$R_f$-Wert: 0,4 (Kieselgelfertigplatten Macherey-Nagel, Polygram, Laufmittel: 3 % Ethanol in Methylenchlorid)

b) 6,7-Dimethoxy-2-(3-brompropyl-1)-1,2,3,4-tetrahydronaphthalin

3,8 g (0,0152 Mol) 3-(6,7-Dimethoxy-1,2,3,4-tetrahydronaphthalin-2-yl)-propanol-1 werden in 40 ml Toluol gelöst. Danach werden 1,57 ml (0,0167 Mol) Phosphortribromid gelöst in 5 ml Toluol innerhalb 30 Minuten zugetropft. Es wird 1 Stunde bei Raumtemperatur nachgerührt. Der Ansatz wird unter Eiskühlung mit Wasser versetzt und anschließend 3 mal mit Essigester extrahiert. Die vereinigten organischen Phasen werden über Magnesiumsulfat getrocknet, abfiltriert und im Vakuum eingedampft.
Ausbeute: 4,4 g (92 % der Theorie),
$R_f$-Wert: 0,9 (Kieselgelfertigplatten, Macherey-Nagel Laufmittel: Methylenchlorid)

Beispiel 1

6,7-Dimethoxy-2-[3-((2-(3,4-dimethoxyphenyl)-ethyl)-methylamino)-propyl]-1-hydroxy-1,2,3,4-tetrahydro-naphthalin

Zu 1,60 g (0,042 Mol) Lithiumaluminiumhydrid in 150 ml trokkenem Tetrahydrofuran werden 9,6 g (0,021 Mol) 3-(6,7-Dimethoxy-1-oxo-1,2,3,4-tetrahydro-naphthalin-2-yl)-N-(2-(3,4-dimethoxy-phenyl)-ethyl)-N-methyl-propionsäureamid, gelöst in 50 ml trockenem Tetrahydrofuran, getropft. Danach wird eine Stunde unter Rückfluß erhitzt, anschließend unter Eiswasserkühlung mit 1,6 ml Wasser, 1,6 ml 15%iger Natronlauge und 5 ml Wasser versetzt. Der Niederschlag wird abgesaugt, mit Tetrahydrofuran nachgewaschen und das Filtrat im Vakuum eingedampft.
Ausbeute: 5,0 g (50 % der Theorie),
Öl, $R_f$-Wert: 0,61 (Aluminiumoxid, Laufmittel: 3 % Ethanol in Methylenchlorid)
Ber.: C 70,40 H 8,41 N 3,16
Gef.: C 70,26 H 8,33 N 3,05

Beispiel 2

6,7-Dimethoxy-2-[3-((2-(3,4-dimethoxyphenyl)-ethyl)-methylamino)-propyl]-1,2,3,4-tetrahydro-naphthalin-hydrochlorid

Zu einer Lösung von 2,73 g (0,006 Mol) 3-(6,7-Dimethoxy-1-oxo-1,2,3,4-tetrahydro-naphthalin-2-yl)-N-(2-(3,4-dimethoxyphenyl)-ethyl)-N-methyl-propionsäureamid in 30 ml absolutem Tetrahydrofuran werden unter Stickstoff 0,74 ml (0,006 Mol) Bortrifluorid-etherat gegeben und anschließend 1,5 ml (0,015 Mol) Borandimethylsulfidkomplex (10 molare Lösung) zugetropft. Anschließend wird 6 Stunden unter Rückfluß erhitzt. Das abgekühlte Reaktionsgemisch wird tropfenweise mit Methanol versetzt. Dann werden 5 ml methanolische Salzsäure zugegeben und weitere 2 Stunden unter Rückfluß erhitzt. Das Methanol und Tetrahydrofuran wird abdestilliert und der Rückstand nach Zugabe von Wasser mit 2 molarer Natronlauge neutralisiert. Der schmierige Niederschlag wird mit Methylenchlorid extrahiert. Die organische Phase wird über Magnesiumsulfat getrocknet, im Vakuum eingedampft und der erhaltene Rückstand über 120 g Aluminiumoxid (neutral, Aktivität II-III) mit Methylenchlorid und anschließenden steigenden Anteilen von Ethanol (bis 0,5 %) gereinigt. Aus einer Lösung in Aceton wird mit methanolischer Salzsäure das Hydrochlorid gefällt.
Ausbeute: 1,24 g (44,6 % der Theorie),
Schmelzpunkt: 143-145°C,
Ber.: C 67,29 H 8,25 N 3,02
Gef.: C 67,16 H 8,18 N 3,07

Beispiel 3

6,7-Dimethoxy-2-[3-((2-(3,4-dimethoxyphenyl)-ethyl)-methylamino)-propyl]-1-oxo-1,2,3,4-tetrahydro-naphthalin-hydrochlorid

Ein Gemisch von 4,43 g (0,01 Mol) 6,7-Dimethoxy-2-[3-((2-(3,4-dimethoxyphenyl)-ethyl)-methylamino)-propyl]-1-hydroxy-1,2,3,4-tetrahydro-naphthalin, 2,80 g (0,025 Mol) Kalium-tert.butylat, 9,10 g (0,05 Mol) Benzophenon und 100 ml Toluol wird 4 Stunden unter Rückfluß erhitzt. Das abgekühlte Reaktionsgemisch wird 2 × mit Wasser gewaschen, dann 3 × mit 1%iger Salzsäure extrahiert. Die salzsauren Phasen werden vereinigt und mit konzentrierter Natronlauge neutralisiert. Der schmierige Niederschlag wird mit Methylenchlorid extrahiert. Die organische Phase wird über Magnesiumsulfat getrocknet, im Vakuum eingedampft und über 300 g Aluminiumoxid (neutral, Aktivität II-III) mit Methylenchlorid und anschließend steigenden Anteilen von Ethanol (bis 0,5 %) gereinigt. Aus einer Lösung in Aceton wird mit methanolischer Salzsäure das Hydrochlorid gefällt.
Ausbeute: 1,7 g (35,6 % der Theorie),
Schmelzpunkt: 160-162°C,
Ber.: C 65,33 H 7,59 N 2,93
Gef.: C 65,40 H 7,50 N 2,77

Beispiel 4

6,7-Dimethoxy-2-[3-((2-(4-benzyloxyphenyl)-ethyl)-methylamino)-propyl]-1,2,3,4-tetrahydro-naphthalin-hydrochlorid

Hergestellt analog Beispiel 2 aus 3-(6,7-Dimethoxy-1-oxo-1,2,3,4-tetrahydronaphthalin-2-yl)-N-(2-(4-benzyloxyphenyl)-ethyl)-N-methyl-propionsäureamid.
Ausbeute: 21 % der Theorie,
Schmelzpunkt: 171-173°C,
Ber.: C 72,99 H 7,90 N 2,75
Gef.: C 73,10 H 8,07 N 2,71

Beispiel 5

6,7-Dimethoxy-2-[3-((2-(4-aminophenyl)-ethyl)-methylamino)-propyl]-1,2,3,4-tetrahydro-naphthalin-dihydrochlorid

Hergestellt analog Beispiel 2 aus 3-(6,7-Dimethoxy-1-oxo-1,2,3,4-tetrahydro-naphthalin-2-yl)-N-(2-(4-aminophenyl)-ethyl)-N-methyl-propionsäureamid.
Ausbeute: 320 mg (23 % der Theorie),
Schmelzpunkt: > 220°C,
Ber.: C 63,29 H 7,97 N 6,15
Gef.: C 63,10 H 7,91 N 6,08

Beispiel 6

6,7-Dimethoxy-2-[3-((2-(4-nitrophenyl)-ethyl)-methylamino)-propyl]-1,2,3,4-tetrahydro-naphthalin

Ein Gemisch von 1,0 g (0,0038 Mol) 6,7-Dimethoxy-2-(3-methylaminopropyl)-1,2,3,4-tetrahydro-naphthalin, 0,87 g (0,038 Mol) 2-(4-Nitrophenyl)ethylbromid und 1,1 ml (0,008 Mol) Triethylamin wird 2 Stunden unter Rückfluß erhitzt. Das abgekühlte Reaktionsgemisch wird in einem Gemisch aus 2 molarer Natronlauge und Methylenchlorid gelöst. Die organische Phase wird abgetrennt, mit Wasser gewaschen, über Magnesiumsulfat getrocknet, im Vakuum eingedampft und über 120 g Aluminiumoxid (neutral, Aktivität II-III) mit Methylenchlorid und anschließend steigenden Anteilen von Ethanol (bis 0,8 %) gereinigt.
Ausbeute: 210 mg (16 % der Theorie),
Öl, $R_f$-Wert: 0,69 (Aluminiumoxid, Laufmittel: 2 % Ethanol in Methylenchlorid)
Ber.: C 69,88 H 7,82 N 6,79
Gef.: C 69,87 H 7,61 N 6,67

Beispiel 7

6,7-Dimethoxy-2-[3-((2-(4-acetaminophenyl)-ethyl)-methylamino)-propyl]-1,2,3,4-tetrahydro-naphthalin-hydrochlorid

0,76 g (0,002 Mol) 6,7-Dimethoxy-2-[3-((2-(4-aminophenyl)- ethyl)-methylamino)-propyl]-1,2,3,4-tetrahydro-naphthalin und 0,3 ml (0,0022 Mol) Triethylamin werden in 10 ml Methylenchlorid gelöst und unter Rühren 0,16 ml (0,0022 Mol) Acetylchlorid zugetropft. Nach 30 Minuten versetzt man mit Wasser. Die wässrige Phase wird 3 × mit Methylenchlorid extrahiert. Die organische Phase wird über Magnesiumsulfat getrocknet und im Vakuum eingedampft. Aus einer Lösung des erhaltenen Rückstandes in Aceton wird mit etherischer Salzsäure das Hydrochlorid gefällt.
Ausbeute: 0,25 g (27 % der Theorie),
Schmelzpunkt: 129-131°C,
Ber.: C 67,73 H 8,09 N 6,08 Cl 7,69
Gef.: C 67,60 H 8,45 N 5,89 Cl 7,73

Beispiel 8

6,7-Dimethoxy-2-[3-((2-(4-hydroxyphenyl)-ethyl)-methylamino)-propyl]-1,2,3,4-tetrahydro-naphthalin

9,1 g (0,019 Mol) 6,7-Dimethoxy-2-[3-((2-(4-benzyloxyphenyl)-ethyl)-methylamino)-propyl]-1,2,3,4-tetrahydro-naphthalin werden in 100 ml Eisessig in Anwesenheit von 1,2 g 10%iger Palladium/Kohle 5 Stunden bei Raumtemperatur und 5 bar Wasserstoff hydriert. Anschließend wird der Katalysators abgesaugt und der Eisessig im Vakuum abdestilliert. Den erhaltenen Rückstand versetzt man mit 10%iger Natronlauge und extrahiert 3 × mit Essigester. Die organische Phase wird über Magnesiumsulfat

getrocknet und im Vakuum eingedampft.
Ausbeute: 6,1 g (83 % der Theorie),
Schmelzpunkt: 90-91°C,
Ber.: C 75,16 H 8,67 N 3,65
Gef.: C 75,00 H 8,79 N 3,73

Beispiel 9

6,7-Dimethoxy-2-[3-((2-(4-methansulfonyloxyphenyl)-ethyl)-methylamino)-propyl]-1,2,3,4-tetrahydro-naphthalin-hydrochlorid

0,96 g (0,0025 Mol) 6,7-Dimethoxy-2-[3-((2-(4-hydroxyphenyl)-ethyl)-methylamino)-propyl]-1,2,3,4-tetrahydro-naphthalin werden in 5 ml Pyridin gelöst und unter Rühren 0,27 ml (0,0035 Mol) Methansulfonsäurechlorid zugetropft. Nach einer Stunde versetzt man mit 10%iger Natronlauge. Die wässrige Phase wird 3 × mit Methylenchlorid extrahiert. Die organische Phase wird über Magnesiumsulfat getrocknet und im Vakuum eingedampft und über 80 g Aluminiumoxid (neutral, Aktivität II-III) mit Methylchlorid gereinigt. Der erhaltene Rückstand wird mit Ether aufgenommen und mit etherischer Salzsäure das Hydrochlorid gefällt.
Ausbeute: 0,38 g (31 % der Theorie),
Schmelzpunkt: 145-146°C,
Ber.: C 60,29 H 7,29 N 2,81 S 6,44 Cl 7,12
Gef.: C 60,35 H 7,38 N 2,85 S 6,27 Cl 7,39

Beispiel 10

6,7-Dimethoxy-2-[3-((2-(4-benzyloxyphenyl)-ethyl)-methylamino)-propyl]-1,2,3,4-tetrahydronaphthalin-hydrochlorid

Hergestellt analog Beispiel 6 aus 6,7-Dimethoxy-2-(3-brompropyl)-1,2,3,4-tetrahydronaphthalin und N-Methyl-(4-benzyloxyphenyl)-ethylamin.
Ausbeute: 43 % der Theorie
Schmelzpunkt: 171-173°C

Beispiel 11

6,7-Dimethoxy-2-[3-((2-(4-trifluormethansulfonyloxyphenyl)-ethyl)-methylamino)-propyl]-1,2,3,4-tetrahydro-naphthalin-hydrochlorid

Hergestellt aus 6,7-Dimethoxy-2-[3-((2-(4-hydroxyphenyl)-ethyl)-methylamino)-propyl]-1,2,3,4-tetrahydro-naphthalin und Trifluormethansulfonsäurechlorid analog Beispiel 9.
Ausbeute: 69 % der Theorie,
Schmelzpunkt: 135-137°C,
Ber.: C 54,39 H 6,03 N 2,54 Cl 6,42S 5,81
Gef.: C 54,34 H 6,28 N 2,74 Cl 6,69S 6,05

Beispiel 12

6,7-Dimethoxy-2-[3-((2-(4-cyanomethoxyphenyl)-ethyl)-methylamino)-propyl]-1,2,3,4-tetrahydro-naphthalin-hydrogentartrat

Hergestellt aus 6,7-Dimethoxy-2-[3-((2-(4-hydroxyphenyl)-ethyl)-methylamino)-propyl]-1,2,3,4-tetrahydro-naphthalin und Chloroacetonitril analog Beispiel 16.
Ausbeute: 31 % der Theorie,
Schmelzpunkt: 63-68°C
Ber.: C 62,92 H 7,04 N 4,89
Gef.: C 62,64 H 7,01 N 4,80

Beispiel 13

6,7-Dimethoxy-2-[3-((2-(4-trifluormethoxyphenyl)-ethyl)-methylamino)-propyl]-1,2,3,4-tetrahydro-naphthalin-hydrochlorid

Hergestellt aus 6,7-Dimethoxy-2-(3-methylaminopropyl)-1,2,3,4-tetrahydro-naphthalin und 2-(4-Trifluormethoxyphenyl)-ethylbromid analog Beispiel 6.
Ausbeute: 50 % der Theorie,

Schmelzpunkt: 124-125°C
Ber.: C 61,53 H 6,82 N 2,87 Cl 7,27
Gef.: C 61,43 H 7,03 N 2,85 Cl 7,55

Beispiel 14

6,7-Dimethoxy-2-[3-((2-(4-methansulfonylaminophenyl)-ethyl)-methylamino)-propyl]-1,2,3,4-tetrahydro-naphthalin-hydrochlorid

Hergestellt aus 6,7-Dimethoxy-2-[3-((2-(4-aminophenyl)-ethyl)-methylamino)-propyl]-1,2,3,4-tetrahydro-naphthalin und Methansulfonsäurechlorid analog Beispiel 7.
Ausbeute: 30 % der Theorie,
Schmelzpunkt: 177-179°C
Ber.: C 60,40 H 7,50 N 5,64
Gef.: C 60,60 H 7,61 N 5,70

Beispiel 15

6,7-Dimethoxy-2-[3-((2-(4-methoxycarbonylaminophenyl)-ethyl)-methylamino)-propyl]-1,2,3,4-tetrahydro-naphthalin-hydrogenfumarat

Hergestellt aus 6,7-Dimethoxy-2-[3-((2-(4-aminophenyl)-ethyl)-methylamino)-propyl]-1,2,3,4-tetrahydro-naphthalin und Chlorameisensäuremethylester analog Beispiel 7.
Ausbeute: 23 % der Theorie,
Schmelzpunkt: 156-160°C (Zers.)
Ber.: C 64,73 H 7,24 N 5,03
Gef.: C 65,03 H 7,08 N 5,20

Beispiel 16

6,7-Dimethoxy-2-[3-((2-(4-ethoxycarbonylmethoxyphenyl)-ethyl)-methylamino)-propyl]-1,2,3,4-tetrahydro-naphthalin-hydrochlorid

Ein Gemisch von 1,15 g (0,003 Mol) 6,7-Dimethoxy-2-[3-((2-(4-hydroxyphenyl)-ethyl)-methylamino)-propyl]-1,2,3,4-tetrahydronaphthalin, 0,34 g (0,003 Mol) Kalium-tert.butylat und 10 ml Dimethylsulfoxid wird 1 Stunde auf 50°C erwärmt. Danach wird 0,5 g (0,003 Mol) Bromessigester zugegeben und nochmals 1 Stunde auf 50°C erwärmt. Das abgekühlte Reaktionsgemisch wird mit Wasser versetzt und mit Essigester extrahiert. Die organische Phase wird über Magnesiumsulfat getrocknet, im Vakuum eingedampft und über 60 g Kieselgel (32-63 µm, Fa. Woelm) mit Methylenchlorid und anschließend steigenden Anteilen von Ethanol (bis 3 %) gereinigt. Aus einer Lösung in Aceton wird mit methanolischer Salzsäure das Hydrochlorid gefällt.
Ausbeute: 0,2 g (13 % der Theorie),
Schmelzpunkt: 134-136°C
Ber.: C 66,45 H 7,97 N 2,77Cl 7,01
Gef.: C 66,31 H 7,91 N 2,74Cl 7,15

Beispiel 17

6,7-Dimethoxy-3-[3-((2-(3,4-dimethoxyphenyl))-ethyl)-methylamino)-propyl]-1,2-dihydro-naphthalin-hydrochlorid

5,0 g (0,011 Mol) 6,7-Dimethoxy-2-[3-((2-(3,4-dimethoxyphenyl)-ethyl)-methylamino)-propyl]-1-hydroxy-1,2,3,4-tetrahydronaphthalin werden in 40 ml Aceton gelöst und mit methanolischer Salzsäure versetzt. Es wird 30 Minuten bei Raumtemperatur gerührt, danach tropfenweise mit Ether versetzt bis eine leichte Trübung auftritt und nochmals 30 Minuten gerührt. Der ausgefallene Niederschlag wird abgesaugt und getrocknet.
Ausbeute: 4,8 g (92 % der Theorie),
Schmelzpunkt: 171-172°C
Ber.: C 67,59 H 7,85 N 3,03
Gef.: C 67,51 H 8,06 N 3,27

Beispiel 18

5,6-Dimethoxy-2-[3-((2-(3,4-dimethoxyphenyl)-ethyl)-methylamino)-propyl]-1-hydroxy-indan

Hergestellt aus 3-(5,6-Dimethoxy-1-oxo-indan-2-yl)-N-(2-(3,4-dimethoxyphenyl)-ethyl)-N-methylpropionsäureamid analog Beispiel 1.
Ausbeute: 58 % der Theorie,
Schmelzpunkt: 83-85°C
Ber.: C 69,90 H 8,11 N 3,26
Gef.: C 69,18 H 8,19 N 3,13

Beispiel 19

5,6-Dimethoxy-2-[3-((2-(3,4-dimethoxyphenyl)-ethyl)-methylamino)-propyl]-1-oxo-indan

Hergestellt aus 5,6-Dimethoxy-2-[3-((2-(3,4-dimethoxyphenyl)-ethyl)-methylamino)-propyl]-1-hydroxy-indan und Benzophenon analog Beispiel 3.
Ausbeute: 16 % der Theorie,
Schmelzpunkt: 98-100°C
Ber.: C 64,71 H 7,39 N 3,02
Gef.: C 64,80 H 7,39 N 2,95

Beispiel 20

6,7-Dimethoxy-2-[3-((2-(4-hydroxycarbonylmethoxyphenyl)-ethyl)-methylamino)-propyl]-1,2,3,4-tetrahydro-naphthalin

Eine Lösung von 700 mg (0,014 Mol) 6,7-Dimethoxy-2-[3-((2-(4-ethoxycarbonylmethoxyphenyl)-ethyl]-methylamino)-propyl]-1,2,3,4-tetrahydro-naphthalin in 10 ml Methanol wird tropfenweise mit 4,1 ml 0,1 n Natronlauge versetzt, anschließend 25 Minuten bei Raumtemperatur gerührt und schließlich mit 4,1 ml 0,1 n Salzsäure versetzt. Die Lösung wird mit Essigester mehrmals extrahiert und nach dem Trocknen der vereinigten organischen Phasen über Magnesiumsulfat im Vakuum eingedampft. Der Rückstand wird in wenig Methylenchlorid aufgenommen, filtriert und das Filtrat zur Trockene eingedampft.
Ausbeute: 240 mg (36 % der Theorie),
Schmelzpunkt:
Ber.: C 70,72 H 7,99 N 3,17
Gef.: C 70,49 H 7,74 N 3,06

Beispiel 21

6,7-Dimethoxy-2-[3-((2-(4-(N,N-bis-methansulfonyl-amino)-phenyl)-ethyl)-methylamino)-propyl]-1,2,3,4-tetrahydronaphthalin-hydrochlorid

Hergestellt aus 6,7-Dimethoxy-2-[3-((2-(4-aminophenyl)-ethyl)-methylamino)-propyl]-1,2,3,4-tetrahydronaphthalin und Methansulfonsäurechlorid analog Beispiel 14.
Ausbeute: 15 % der Theorie,
Schmelzpunkt: 106-108°C (Zers.)
Ber.: C 54,29 H 6,83 N 4,87
Gef.: C 54,10 H 6,77 N 4,62

Beispiel 22

6,7-Dimethoxy-2-[3-((3-(4-bromphenyl)-propyl)-methylamino)-propyl]-1,2,3,4-tetrahydronaphthalin-hydrogenfumarat

Hergestellt aus 6,7-Dimethoxy-2-(3-methylamino-propyl)-1,2,3,4-tetrahydronaphthalin und 3-(4-bromphenyl)-1-brompropan analog Beispiel 6.
Ausbeute: 35 % der Theorie,
Schmelzpunkt: 59°C
Ber.: C 60,42 H 6,65 N 2,43
Gef.: C 60,10 H 6,43 N 2,40

Beispiel 23

6,7-Dimethoxy-2-[3-((3-(4-cyanophenyl)-propyl)-methylamino)-propyl]-1,2,3,4-tetrahydronaphthalin-hydrogenfumarat

Hergestellt aus 6,7-Dimethoxy-2-(3-methylamino-propyl)-1,2,3,4-tetrahydronaphthalin und 3-(4-Cyano-phenyl)-1-brompropan analog Beispiel 6.
Ausbeute: 58 % der Theorie,
Schmelzpunkt: ab 57°C Zersetzung
Ber.: C 68,94 H 7,33 N 5,36
Gef.: C 68,71 H 7,15 N 5,20

Beispiel 24

6,7-Dimethoxy-2-[3-((2-(4-methylsulfonylphenyl)-ethyl)-methylamino)-propyl]-1,2,3,4-tetrahydronaphthalin

Hergestellt aus 6,7-Dimethoxy-2-(3-methylamino-propyl)-1,2,3,4-tetrahydronaphthalin und 2-(4-Methylsulfonylphenyl)-1-bromethan analog Beispiel 6.
Ausbeute: 10,4 % der Theorie,
Öl, $R_f$-Wert: 0,63 (Aluminiumoxid, Laufmittel: 3 % Ethanol in Methylenchlorid)
Ber.: C 67,32 H 7,85 N 3,14
Gef.: C 67,14 H 7,98 N 3,25

Beispiel 25

6,7-Dimethoxy-2-[3-((3-(4-methoxyphenyl)-propyl)-methylamino)-propyl]-1,2,3,4-tetrahydronaphthalin

Hergestellt aus 6,7-Dimethoxy-2-(3-methylamino-propyl)- 1,2,3,4-tetrahydronaphthalin und 3-(4-Methoxyphenyl)-1-brompropan analog Beispiel 6.
Ausbeute: 68,7 % der Theorie,
Schmelzpunkt: 39°C
Ber.: C 75,87 H 8,82 N 3,40
Gef.: C 75,69 H 8,94 N 3,59

Beispiel 26

6,7-Dimethoxy-2-[3-((2-phenyl-ethyl)-methylamino)-propyl]-1,2,3,4-tetrahydronaphthalin

Hergestellt aus 2-Phenyl-1-chlorethan und 6,7-Dimethoxy-2-(3-methylamino-propyl)-1,2,3,4-tetrahydronaphthalin analog Beispiel 6.
Ausbeute: 42 % der Theorie,
Öl, $R_f$-Wert: 0,42 (Aluminiumoxid, Laufmittel: 2 % Ethanol in Methylenchlorid)
Ber.: C 78,43 H 9,05 N 3,81
Gef.: C 78,63 H 9,29 N 3,66

Beispiel 27

5,6-Dimethoxy-2-[3-((4-benzyloxyphenyl)-ethyl)-methylamino)-propyl]-1-hydroxy-indan

Hergestellt aus 3-(5,6-Dimethoxy-1-oxo-indan-2-yl)-N-(2-(4-benzyloxyphenyl)-ethyl)-N-methyl-propionsäureamid und Lithiumaluminiumhydrid analog Beispiel 1.
Ausbeute: 90 % der Theorie,
Schmelzpunkt: 80-83°C
Ber.: C 75,76 H 7,84 N 2,95
Gef.: C 75,58 H 8,01 N 2,79

Beispiel 28

6,7-Dimethoxy-2-[3-((2-(4-(2-hydroxyethoxy)-phenyl)-ethyl)-methylamino)-propyl]-1,2,3,4-tetrahydronaphthalin-hydrochlorid

Hergestellt aus 6,7-Dimethoxy-2-[3-((2-(4-ethoxycarbonylmethoxyphenyl)-ethyl)-methylamino)-propyl]-1,2,3,4-tetrahydronaphthalin und Lithiumaluminiumhydrid analog Beispiel 1.

Ausbeute: 43 % der Theorie,
Schmelzpunkt: 141-143°C
Ber.: C 67,30 H 8,25 N 3,02 Cl 7,64
Gef.: C 67,20 H 8,28 N 3,04 Cl 7,65

Beispiel 29

6,7-Dimethoxy-2-[3-((2-(4-(2-benzyloxyphenyl)-ethyl)-methylamino)-propyl]-1-hydroxy-1,2,3,4-tetrahydronaphthalin

Hergestellt aus 3-(6,7-Dimethoxy-1-oxo-1,2,3,4-tetrahydronaphthalin-2-yl)-N-(2-(4-benzyloxyphenyl)-ethyl)-N-methylpropionsäureamid und Lithiumaluminiumhydrid analog Beispiel 1.
Ausbeute: 53,6 % der Theorie,
Schmelzpunkt: 75°C
Ber.: C 70,77 H 7,66 N 2,66
Gef.: C 71,00 H 7,52 N 2,56

Beispiel 30

6,7-Dimethoxy-2-[3-((2-(4-hydroxyphenyl)-ethyl)-methylamino)-propyl]-1-hydroxy-1,2,3,4-tetrahydronaphthalin

Hergestellt aus 6,7-Dimethoxy-2-[3-((2-(4-benzyloxy-phenyl)-ethyl)-methylamino)-propyl]-1-hydroxy-1,2,3,4-tetrahydronaphthalin und Wasserstoff analog Beispiel 8.
Ausbeute: 47,5 % der Theorie,
Schmelzpunkt: 97°C
Ber.: C 72,15 H 8,33 N 3,51
Gef.: C 72,14 H 8,43 N 3,53

Beispiel 31

6,7-Dimethoxy-2-[3-((2-(4-methansulfonyloxy-phenyl)-ethyl)-methylamino)-propyl]-1-hydroxy-1,2,3,4-tetrahydronaphthalin

Hergestellt aus 6,7-Dimethoxy-2-[3-((2-(4-hydroxy-phenyl)-ethyl)-methylamino)-propyl]-1-hydroxy-1,2,3,4-tetrahydro-naphthalin und Methansulfonsäurechlorid analog Beispiel 9.
Ausbeute: 16,7 % der Theorie,
Schmelzpunkt:
Ber.: C 62,87 H 7,39 N 2,93 Cl 6,71
Gef.: C 62,05 H 7,50 N 2,75 Cl 6,88

Beispiel 32

6,7-Dimethoxy-2-[3-((2-(4-trifluormethansulfonyloxy-phenyl)-ethyl)-methylamino)-propyl]-1-hydroxy-1,2,3,4-tetrahydronaphthalin

Hergestellt aus 6,7-Dimethoxy-2-[3-((2-(4-hydroxy-phenyl)-ethyl)-methylamino)-propyl]-1-hydroxy-1,2,3,4-tetrahydronaphthalin und Trifluormethansulfonsäurechlorid analog Beispiel 9.
Ausbeute: 54,8 % der Theorie,
Schmelzpunkt: 119°C
Ber.: C 56,49 H 6,07 N 2,63 Cl 6,03
Gef.: C 56,36 H 6,01 N 2,49 Cl 6,86

Beispiel 33

6,7-Dimethoxy-2-[3-((2-(4-ethoxycarbonylmethoxy-phenyl)-ethyl)-methylamino)-propyl]-1-hydroxy-1,2,3,4-tetrahydronaphthalin

Hergestellt aus 6,7-Dimethoxy-2-[3-((2-(4-hydroxy-phenyl)-ethyl)-methylamino)-propyl]-1-hydroxy-1,2,3,4-tetrahydronaphthalin und Bromessigsäureethylester analog Beispiel 9.
Ausbeute: 29 % der Theorie,
Öl, $R_f$-Wert 0,2 (Aluminiumoxid, Laufmittel: 3 % Ethanol in Methylenchlorid)
Ber.: C 69,25 H 8,09 N 2,88
Gef.: C 69,00 H 7,93 N 2,63

Beispiel 34

6,7-Dimethoxy-2-[3-((2-(4-hydroxycarbonylmethoxy-phenyl)-ethyl)-methylamino)-propyl]-1-hydroxy-1,2,3,4-tetrahydronaphthalin

Hergestellt aus 6,7-Dimethoxy-2-[3-((2-(4-hydroxy-phenyl)-ethyl)-methylamino)-propyl]-1-hydroxy-1,2,3,4-tetrahydronaphthalin und Bromessigsäureethylester/Natronlauge analog Beispiel 20.
Ausbeute: 37 % der Theorie,
Schmelzpunkt: 92°C
Ber.: C 68,25 H 7,71 N 3,06
Gef.: C 68,58 H 7,63 N 2,92

Beispiel 35

6,7-Dimethoxy-2-[3-((2-(4-benzyloxyphenyl)-ethyl)-methylamino)-propyl]-1-oxo-1,2,3,4-tetrahydronaphthalin-hydrochlorid

Hergestellt aus 6,7-Dimethoxy-2-[3-((2-(4-benzyloxy-phenyl)-ethyl)-methylamino)-propyl]-1-hydroxy-1,2,3,4-tetrahydronaphthalin und Kalium-tert.butylat/Benzophenon analog Beispiel 3.
Ausbeute: 26 % der Theorie,
Schmelzpunkt: 191°C
Ber.: C 71,04 H 7,31 N 2,67 Cl 6,76
Gef.: C 71,12 H 7,24 N 2,61 Cl 6,87

Beispiel 36

6,7-Dimethoxy-2-[3-((2-(4-benzyloxyphenyl)-ethyl)-methylamino)-propyl]-3,4-dihydronaphthalin-hydrochlorid

Hergestellt aus 6,7-Dimethoxy-2-[3-((2-(4-benzyloxy-phenyl)-ethyl)-methylamino)-propyl]-1-hydroxy-1,2,3,4-tetrahydronaphthalin und Salzsäure analog Beispiel 17.
Ausbeute: 18,6 % der Theorie,
Schmelzpunkt: 185°C
Ber.: C 73,28 H 7,54 N 2,76 Cl 6,98
Gef.: C 73,66 H 7,20 N 2,78 Cl 7,43

Beispiel 37

6,7-Dimethoxy-2-[3-((2-(4-hydroxyphenyl)-ethyl)-methylamino)-propyl]-3,4-dihydronaphthalin

Hergestellt aus 6,7-Dimethoxy-2-[3-((2-(4-hydroxy-phenyl)-ethyl)-methylamino)-propyl]-1-hydroxy-1,2,3,4-tetrahydronaphthalin und Salzsäure analog Beispiel 17.
Ausbeute: 65 % der Theorie,
Öl, $R_f$-Wert: 0,83 (Aluminiumoxid, Laufmittel: 5 % Ethanol in Methylenchlorid)
Ber.: C 75,56 H 8,19 N 3,67
Gef.: C 75,29 H 8,10 N 3,62

Beispiel 38

6,7-Dimethoxy-2-[3-((2-(4-methansulfonyloxyphenyl)-ethyl)-methylamino)-propyl]-3,4-dihydronaphthalin-hydrochlorid

Hergestellt aus 6,7-Dimethoxy-2-[3-((2-(4-methansulfonyloxyphenyl)-ethyl)-methylamino)-propyl]-1-hydroxy-1,2,3,4-tetrahydronaphthalin und Salzsäure analog Beispiel 17.
Ausbeute: 42,8 % der Theorie,
Schmelzpunkt: 81°C (sintert ab 59°C)
Ber.: C 58,41H 7,06N 2,72Cl 6,89S 6,24
Gef.: C 58,60H 7,07N 2,54Cl 7,00S 6,37

Beispiel 39

6,7-Dimethoxy-2-[3-((2-(4-trifluormethansulfonyloxyphenyl)-ethyl)-methylamino)-propyl]-3,4-dihydronaphthalin

Hergestellt aus 6,7-Dimethoxy-2-[3-((2-(4-trifluormethansulfonyloxyphenyl)-ethyl)-methylamino)-propyl]-1-hydroxy-1,2,3,4-tetrahydronaphthalin und Salzsäure analog Beispiel 17.
Ausbeute: 77 % der Theorie,
Öl, $R_f$-Wert: 0,92 (Aluminiumoxid, Laufmittel: 3 % Ethanol in Methylenchlorid)
Ber.: C 58,47 H 5,89 N 2,73 Cl 6,24
Gef.: C 58,61 H 5,88 N 2,54 Cl 6,75

Beispiel 40

5,6-Dimethoxy-2-[3-((2-(4-methoxyphenyl)-ethyl)-methylamino)-propyl]-1-hydroxy-indan-oxalat

Hergestellt aus 3-(5,6-Dimethoxy-1-oxo-indan-2-yl)-N-(2-(4-methoxyphenyl)-ethyl)-N-methylpropionsäureamid und Lithiumaluminiumhydrid analog Beispiel 1.
Ausbeute: 90 % der Theorie,
Schmelzpunkt: 80-83°C
Ber.: C 63,79H 7,21N 2,86
Gef.: C 63,55H 7,31N 3,02

Beispiel 41

5,6-Dimethoxy-2-[3-((2-(4-methoxyphenyl)-ethyl)-methylamino)-propyl]-1-oxo-indan-hydrochlorid

Hergestellt aus 5,6-Dimethoxy-2-[3-((2-(4-methoxyphenyl)-ethyl)-methylamino)-propyl]-1-hydroxy-indan und Benzophenon analog Beispiel 13.
Ausbeute: 21 % der Theorie,
Schmelzpunkt: 193-194°C
Ber.: C 66,42 H 7,43 N 3,23 Cl 8,17
Gef.: C 66,30 H 7,58 N 3,39 Cl 8,30

Beispiel 42

5,6-Dimethoxy-2-[3-((2-(4-benzyloxyphenyl)-ethyl)-methylamino)-propyl]-1-hydroxy-indan

Hergestellt aus 3-(5,6-Dimethoxy-1-oxo-indan-2-yl)-N-(2-(4- benzyloxyphenyl)-ethyl)-N-methylpropionsäureamid und Lithiumaluminiumhydrid analog Beispiel 1.
Ausbeute: 90 % der Theorie,
Schmelzpunkt: 80-83°C
Ber.: C 75,76 H 7,84 N 2,95
Gef.: C 75,58 H 8,07 N 2,79

Beispiel 43

5,6-Dimethoxy-2-[3-((2-(4-benzyloxyphenyl)-ethyl)-methylamino)-propyl]-1-oxo-indan-hydrochlorid

Hergestellt aus 5,6-Dimethoxy-2-[3-((2-(4-benzyloxyphenyl)-ethyl)-methylamino)-propyl]-1-hydroxy-indan und Benzophenon analog Beispiel 3.
Ausbeute: 12 % der Theorie,
Schmelzpunkt: 189-191°C
Ber.: C 70,64 H 7,11 N 2,75Cl 6,95
Gef.: C 70,50 H 7,22 N 2,83Cl 7,20

Beispiel 44

5,6-Dimethoxy-2-[3-((2-(4-methylphenyl)-ethyl)-methylamino)-propyl]-1-hydroxy-indan

Hergestellt aus 3-(5,6-Dimethoxy-1-oxo-indan-2-yl)-N-(2-(4-methylphenyl)-ethyl)-N-methylpropionsäureamid und Lithiumaluminiumhydrid analog Beispiel 1.
Ausbeute: 83 % der Theorie,
Schmelzpunkt: 89-90°C

Ber.: C 75,16 H 8,67 N 3,65
Gef.: C 74,96 H 8,65 N 3,58

Beispiel 45

5,6-Dimethoxy-2-[3-((2-(4-methylphenyl)-ethyl)-methylamino)-propyl]-1-oxo-indan-hydrochlorid

Hergestellt aus 5,6-Dimethoxy-2-[3-((2-(4-methylphenyl)-ethyl)-methylamino)-propyl]-1-hydroxy-indan und Benzophenon analog Beispiel 3.
Ausbeute: 19 % der Theorie,
Schmelzpunkt: 193-194°C
Ber.: C 68,96 H 7,72 N 3,35 Cl 8,48
Gef.: C 68,86 H 7,72 N 3,45 Cl 8,63

Beispiel 46

4-Methyl-2-[3-((2-(3,5-dimethoxyphenyl)-ethyl)-methylamino)-propyl]-1-hydroxy-indan-oxalat

Hergestellt aus 3-(4-Methyl-1-oxo-indan-2-yl)-N-(2-(3,5-dimethoxy-phenyl)-ethyl)-N-methylpropionsäureamid und Lithiumaluminiumhydrid analog Beispiel 1.
Ausbeute: 90 % der Theorie,
Schmelzpunkt: 82-83°C
Ber.: C 65,94 H 7,45 N 2,96
Gef.: C 66,03 H 7,55 N 3,05

Beispiel 47

4-Methyl-2-[3-((2-(3,5-dimethoxyphenyl)-ethyl)-methylamino)-propyl]-1-oxo-indan

Hergestellt aus 4-Methyl-2-[3-((2-(3,5-dimethoxyphenyl)- ethyl)-methylamino)-propyl]-l-hydroxy-indan und Benzophenon analog Beispiel 3.

Beispiel 48

4-Methyl-2-[3-((2-phenylethyl)-methylamino)-propyl]-1-hydroxy-indan-oxalat

Hergestellt aus 3-(4-Methyl-1-oxo-indan-2-yl)-N-(2-phenylethyl)-N-methylpropionsäureamid und Lithiumaluminiumhydrid analog Beispiel 1.
Ausbeute: 90 % der Theorie,
Schmelzpunkt: 88-90°C
Ber.: C 69,71 H 7,56 N 3,39
Gef.: C 69,57 H 7,63 N 3,31

Beispiel 49

4-Methyl-2-[3-((2-phenyl-ethyl)-methylamino)-propyl]-3-oxo-indan-fumarat

Hergestellt aus 4-Methyl-2-[3-((2-phenyl-ethyl)-methylamino)-propyl]-1-hydroxy-indan und Benzophenon analog Beispiel 3.
Ausbeute: 5 % der Theorie,
Schmelzpunkt: 118-121°C
Ber.: C 71,37 H 7,14 N 3,20
Gef.: C 71,19 H 7,33 N 3,31

Beispiel 50

4-Methyl-2-[3-((2-(3,4,5-trimethoxyphenyl)-ethyl)-methylamino)-propyl]-1-hydroxy-indan

Hergestellt aus 3-(4-Methyl-1-oxo-indan-2-yl)-N-(2-(3,4,5-trimethoxyphenyl)-ethyl)-N-methylpropionsäureamid und Lithiumaluminiumhydrid analog Beispiel 1.
Ausbeute: 85 % der Theorie,
Schmelzpunkt: 75-77°C
Ber.: C 64,40 H 7,41 N 2,78
Gef.: C 64,49 H 7,38 N 3,09

Beispiel 51

4-Methyl-2-[3-((2-(3,4,5-trimethoxyphenyl)-ethyl)-methylamino)-propyl]-1-oxo-indan

Hergestellt aus 4-Methyl-2-[3-((2-(3,4,5-trimethoxy-phenyl)-ethyl)-methylamino)-propyl]-1-hydroxy-indan und Benzophenon analog Beispiel 3.

Beispiel 52

5,6-Dimethoxy-2-[3-((2-(4-benzyloxyphenyl)-ethyl)-methylamino)-propyl]-inden-hydrochlorid

Hergestellt aus 5,6-Dimethoxy-2-[3-((2-(4-benzyloxyphenyl)-ethyl)-methylamino)-propyl]-1-hydroxy-indan und Salzsäure analog Beispiel 17.
Ausbeute: 88 % der Theorie,
Schmelzpunkt: 181-183°C
Ber.: C 72,93 H 7,34 N 2,84 Cl 7,18
Gef.: C 72,50 H 7,39 N 2,62 Cl 7,32

Beispiel 53

5,6-Dimethoxy-2-[3-((2-(4-methoxyphenyl)-ethyl)-methylamino)-propyl]-inden-hydrochlorid

Hergestellt aus 5,6-Dimethoxy-2-[3-((2-(4-methoxyphenyl)-ethyl)-methylamino)-propyl]-1-hydroxy-indan und Salzsäure analog Beispiel 17.
Ausbeute: 68 % der Theorie,
Schmelzpunkt: 188-190°C
Ber.: C 68,96 H 7,72 N 3,35 Cl 8,48
Gef.: C 68,86 H 7,72 N 3,25 Cl 8,68

Beispiel 54

5,6-Dimethoxy-2-[3-((2-(4-methylphenyl)-ethyl)-methylamino)-propyl]-inden-hydrochlorid

Hergestellt aus 5,6-Dimethoxy-2-[3-((2-(4-methylphenyl)-ethyl)-methylamino)-propyl]-1-hydroxy-indan und Salzsäure analog Beispiel 17.
Ausbeute: 72 % der Theorie,
Schmelzpunkt: 205-207°C
Ber.: C 71,71 H 8,02 N 3,48 Cl 8,82
Gef.: C 71,63 H 8,04 N 3,48 Cl 8,94

Beispiel 55

7-Methyl-2-[3-((2-(3,5-dimethoxyphenyl)-ethyl)-methylamino)-propyl]-inden-oxalat

Hergestellt aus 4-Methyl-2-[3-((2-(3,5-dimethoxyphenyl)-ethyl)-methylamino)-propyl]-1-hydroxy-indan und Salzsäure analog Beispiel 17.
Ausbeute: 37 % der Theorie,
Schmelzpunkt: 133-134°C
Ber.: C 68,55 H 7,30 N 3,07
Gef.: C 68,43 H 7,45 N 3,17

Beispiel 56

7-Methyl-2-[3-((2-phenylethyl)-methylamino)-propyl]-inden-fumarat

Hergestellt aus 4-Methyl-2-[3-((2-phenylethyl)-methylamino)-propyl]-1-hydroxy-indan und Salzsäure analog Beispiel 17.
Ausbeute: 33 % der Theorie,
Schmelzpunkt: 124-126°C
Ber.: C 74,08 H 7,41 N 3,32
Gef.: C 73,83 H 7,38 N 3,52

Beispiel 57

7-Methyl-2-[3-((2-(3,4,5-trimethoxyphenyl)ethyl)-methylamino)-propyl]-inden-oxalat

Hergestellt aus 4-Methyl-2-[3-((2-(3,4,5-trimethoxyphenyl)-ethyl)-methylamino)-propyl]-1-hydroxy-indan und Salzsäure analog Beispiel 17.
Ausbeute: 28 % der Theorie,
Schmelzpunkt: 102-104°C (Zers.)
Ber.: C 66,78 H 7,27 N 2,88
Gef.: C 66,59 H 7,48 N 2,62

Beispiel 58

5,6-Dimethoxy-2-[3-((2-(4-benzyloxyphenyl)-ethyl)-methylamino)-propyl]-1-oxo-indan-hydrochlorid

Hergestellt aus 5,6-Dimethoxy-2-[3-((2-(4-benzyloxyphenyl)-ethyl)-methylamino)-propyl]-1-hydroxy-indan und Benzophenon analog Beispiel 3.
Ausbeute: 12 % der Theorie,
Schmelzpunkt: 189-191°C
Ber.: C 70,64 H 7,11 N 2,75 Cl 6,95
Gef.: C 70,50 H 7,22 N 2,83 Cl 7,10

Beispiel 59

6,7-Dimethoxy-3-[3-((2-(3-benzyloxyphenyl)-ethyl)-methylamino)-propyl]-1,2-dihydro-naphthalin-hydrochlorid

Hergestellt aus 6,7-Dimethoxy-2-[3-((2-(3-benzyloxyphenyl)-ethyl)-methylamino)-propyl]-1,2,3,4-tetrahydro-1-hydroxy-naphthalin und Salzsäure analog Beispiel 17.
Ausbeute: 23 % der Theorie,
Schmelzpunkt: 133-134°C
Ber.: C 73,28 H 7,54 N 2,76 Cl 6,98
Gef.: C 73,12 H 7,46 N 2,89 Cl 7,07

Beispiel 60

6,7-Dimethoxy-2-[3-((2-(4-methansulfonyloxyphenyl)-ethyl)-methylamino)-propyl]-1-oxo-1,2,3,4-tetrahydro-naphthalin-hydrochlorid

Hergestellt aus 6,7-Dimethoxy-2-[3-((2-(4-methansulfonyloxyphenyl)-ethyl)-methylamino)-propyl]-1-hydroxy-1,2,3,4-tetrahydronaphthalin und Benzophenon analog Beispiel 3.
Ausbeute: 53 % der Theorie,
Schmelzpunkt: 183°C
Ber.: C 58,64 H 6,69 N 2,73 Cl 6,92S 6,26
Gef.: C 58,48 H 6,93 N 2,68 Cl 7,22S 6,50

Beispiel 61

6,7-Dimethoxy-2-[3-((2-(3-methansulfonyloxyphenyl)-ethyl)-methylamino)-propyl]-1-oxo-1,2,3,4-tetrahydro-naphthalin-hydrochlorid

Hergestellt aus 6,7-Dimethoxy-2-[3-((2-(3-methansulfonyloxyphenyl)-ethyl)-methylamino)-propyl]-1-hydroxy-1,2,3,4-tetrahydronaphthalin und Benzophenon analog Beispiel 3.
Ausbeute: 28 % der Theorie,
Schmelzpunkt: 81°C (sint. ab 66°C)
Ber.: C 58,64 H 6,69 N 2,74
Gef.: C 58,56 H 6,83 N 2,69

Beispiel 62

6,7-Dimethoxy-2-[3-((2-(3-benzyloxyphenyl)-ethyl)-methylamino)-propyl]-1-oxo-1,2,3,4-tetrahydro-naphthalin-hydrochlorid

Hergestellt aus 6,7-Dimethoxy-2-[3-((2-(3-benzyloxyphenyl)-ethyl)-methylamino)-propyl]-1-hydroxy-1,2,3,4-tetrahydronaphthalin und Benzophenon analog Beispiel 3.

Ausbeute: 27 % der Theorie,
Schmelzpunkt: 170°C
Ber.: C 71,04 H 7,31 N 2,67 Cl 6,77
Gef.: C 70,20 H 7,31 N 2,64 Cl 6,68

Beispiel 63

6,7-Dimethoxy-2-[3-((2-(3-hydroxyphenyl)-ethyl)-methylamino)-propyl]-1-hydroxy-1,2,3,4-tetrahydro-naphthalin-oxalat

Hergestellt aus 6,7-Dimethoxy-2-[3-((2-(3-benzyloxyphenyl)-ethyl)-methylamino)-propyl]-1-hydroxy-1,2,3,4-tetrahydronaphthalin und Wasserstoff in Gegenwart von Palladium/Kohle analog Beispiel 8.
Ausbeute: 84 % der Theorie,
Schmelzpunkt: 77°C
Ber.: C 63,79 H 7,21 N 2,86
Gef.: C 63,57 H 7,40 N 2,68

Beispiel 64

6,7-Dimethoxy-2-[3-((2-(3-hydroxyphenyl)-ethyl)-methylamino)-propyl]-1,2,3,4-tetrahydro-naphthalin-hydrochlorid

Hergestellt aus 6,7-Dimethoxy-3-[3-((2-(3-benzyloxyphenyl)-ethyl)-methylamino)-propyl]-1,2-dihydronaphthalin und Wasserstoff in Gegenwart von Palladium/Kohle in Ethanol als Lösungsmittel analog Beispiel 8.
Ausbeute: 65 % der Theorie,
Schmelzpunkt: sint. ab 72°C
Ber.: C 68,64 H 8,16 N 3,34 Cl 8,44
Gef.: C 68,50 H 7,95 N 3,02 Cl 8,84

Beispiel 65

6,7-Dimethoxy-2-[3-((2-(3-benzyloxyphenyl)-ethyl)-methylamino)-propyl]-1-hydroxy-1,2,3,4-tetrahydro-naphthalin-oxalat

Hergestellt aus 3-(6,7-Dimethoxy-1-oxo-1,2,3,4-tetrahydro-naphthalin-2-yl)-N-(2-(3-benzyloxyphenyl)-ethyl)-N-methylpropionsäureamid und Lithiumaluminiumhydrid analog Beispiel 1
Ausbeute: 51 % der Theorie,
Schmelzpunkt: 108°C (Zers.)
Ber.: C 68,37 H 7,13 N 2,42
Gef.: C 68,09 H 7,08 N 2,38

Beispiel I

Tabletten zu 10 mg 6,7-Dimethoxy-2-[3-((2-(3,4-dimethoxyphenyl)-ethyl)-methylamino)-propyl]-1-oxo-1,2,3,4-tetrahydro-naphthalin

Zusammensetzung:
1 Tablette enthält:

| | |
|---|---|
| Wirksubstanz | 10,0 mg |
| Maisstärke | 57,0 mg |
| Milchzucker | 48,0 mg |
| Polyvinylpyrrolidon | 4,0 mg |
| Magnesiumstearat | 1,0 mg |
| | 120,0 mg |

Herstellungsverfahren

Der Wirkstoff, Maisstärke, Milchzucker und Polyvinylpyrrolidon werden gemischt und mit Wasser befeuchtet. Die feuchte Mischung wird durch ein Sieb mit 1,5 mm-Maschenweite gedrückt und bei ca. 45°C getrocknet. Das trockene Granulat wird durch ein Sieb mit 1,0 mm-Maschenweite geschlagen und mit Magnesiumstearat vermischt. Die fertige Mischung preßt man auf einer Tablettenpresse mit Stempeln von 7 mm Durchmesser, die mit einer Teilkerbe versehen sind, zu Tabletten.
Tablettengewicht: 120 mg

Beispiel II

Dragées zu 5 mg 6,7-Dimethoxy-2-[3-((2-(3,4-dimethoxyphenyl)-ethyl)-methylamino)-propyl]-1-oxo-1,2,3,4-tetrahydro-naphthalin

1 Dragéekern enthält:

| | |
|---|---|
| Wirksubstanz | 5,0 mg |
| Maisstärke | 41,5 mg |
| Milchzucker | 30,0 mg |
| Polyvinylpyrrolidon | 3,0 mg |
| Magnesiumstearat | 0,5 mg |
| | 80,0 mg |

Herstellungsverfahren

Der Wirkstoff, Maisstärke, Milchzucker und Polyvinylpyrrolidon werden gut gemischt und mit Wasser befeuchtet. Die feuchte Masse drückt man durch ein Sieb mit 1 mm-Maschenweite, trocknet bei ca. 45°C und schlägt das Granulat anschließend durch dasselbe Sieb. Nach dem Zumischen von Magnesiumstearat werden auf einer Tablettiermaschine gewölbte Dragéekerne mit einem Durchmesser von 6 mm gepreßt. Die so hergestellten Dragéekerne werden auf bekannte Weise mit einer Schicht überzogen, die im wesentlichen aus Zucker und Talkum besteht. Die fertigen Dragées werden mit Wachs poliert.
Dragéegewicht: 130 mg

Beispiel III

Ampullen zu 5 mg 6,7-Dimethoxy-2-[3-((2-(3,4-dimethoxyphenyl)-ethyl)-methylamino)-propyl]-1-oxo-1,2,3,4-tetrahydro-naphthalin

1 Ampulle enthält:

| | |
|---|---|
| Wirksubstanz | 5,0 mg |
| Sorbit | 50,0 mg |
| Wasser für Injektionszwecke ad | 2,0 ml |

Herstellungsverfahren

In einem geeigneten Ansatzgefäß wird der Wirkstoff in Wasser für Injektionszwecke gelöst und die Lösung mit Sorbit isotonisch gestellt.

Nach Filtration über einem Membranfilter wird die Lösung unter $N_2$-Begasung in gereinigte und sterilisierte Ampullen abgefüllt und 20 Minuten im strömenden Wasserdampf autoklaviert.

Beispiel IV

Suppositorien zu 15 mg 6,7-Dimethoxy-2-[3-((2-(3,4-dimethoxyphenyl)-ethyl)-methylamino)-propyl]-1-oxo-1,2,3,4-tetrahydronaphthalin

1 Zäpfchen enthält:

| | |
|---|---|
| Wirksubstanz | 0,015 g |
| Hartfett (z.B. Witepsol H 19 und W 45) | 1,685 g |
| | 1,700 g |

Herstellungsverfahren:

Das Hartfett wird geschmolzen. Bei 38°C wird die gemahlene Wirksubstanz in der Schmelze homogen dispergiert. Es wird auf 35°C abgekühlt und in schwach vorgekühlte Suppositorienformen ausgegossen.

Beispiel V

Tropfenlösung mit 10 mg 6,7-Dimethoxy-2-[3-((2-(3,4-dimethoxyphenyl)-ethyl)-methylamino)-propyl]-1-oxo-1,2,3,4-tetrahydronaphthalin

100 ml Lösungen enthalten:

| | | |
|---|---|---|
| Wirksubstanz | | 0,2 g |
| Hydroxyäthylcellulose | | 0,15 g |
| Weinsäure | | 0,1 g |
| Sorbitlösung 70 % Trockensubstanz | | 30,0 g |
| Glycerin | | 10,0 g |
| Benzoesäure | | 0,15 g |
| Dest.Wasser | ad | 100 ml |

Herstellungsverfahren:

Dest.Wasser wird auf 70°C erhitzt. Hierin wird unter Rühren Hydroxyäthylcellulose, Benzoesäure und Weinsäure gelöst. Es wird auf Raumtemperatur abgekühlt und hierbei das Glycerin und die Sorbitlösung unter Rühren zugegeben. Bei Raumtemperatur wird der Wirkstoff zugegeben und bis zur völligen Auflösung gerührt. Anschließend wird zur Entlüftung des Saftes unter Rühren evakuiert.

## Patentansprüche

1. Neue Naphthalin- und Indanderivate der Formel

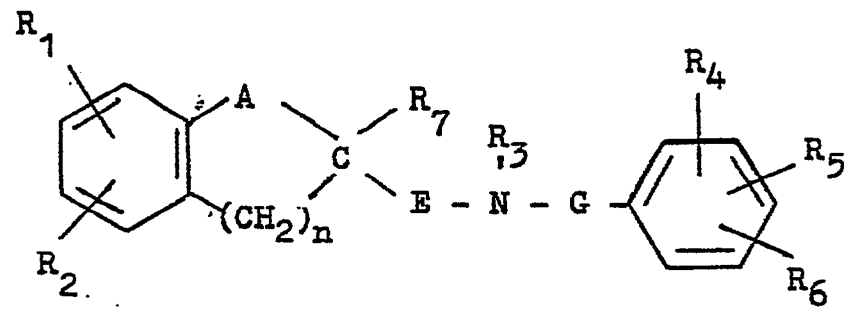

(I)

in der

n die Zahl 1 oder 2,

A eine Carbonylgruppe und $R_7$ ein Wasserstoftatom oder

A eine Gruppe der Formel

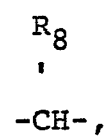

in welcher
$R_8$ ein Wasserstoffatom, eine Hydroxy-, Alkanoyloxy- oder Alkoxycarbonyloxygruppe darstellt, und $R_7$ ein Wasserstoffatom oder $R_7$ und $R_8$ zusammen eine weitere Bindung darstellen,

E eine gegebenenfalls durch eine Alkylgruppe substituierte geradkettige Alkylengruppe mit 3 oder 4 Kohlenstoffatomen,

G eine gegebenenfalls durch eine Alkylgruppe substituierte geradkettige Alkylengruppe mit 2 bis 5 Kohlenstoffatomen,

$R_1$ ein Wasserstoff- oder Halogenatom, eine Trifluormethyl-, Nitro-, Amino-, Alkylamino-, Dialkylamino-, Alkyl-, Hydroxy-, Alkoxy- oder Phenylalkoxygruppe,

$R_2$ ein Wasserstoffatom oder Halogenatom, eine Hydroxy-, Alkoxy-, Phenylalkoxy- oder Alkylgruppe, oder

$R_1$ und $R_2$ zusammen eine Alkylendioxygruppe mit 1 oder 2 Kohlenstoffatomen,

$R_3$ ein Wasserstoffatom, eine Alkylgruppe oder eine Alkenylgruppe mit 3 bis 5 Kohlenstoffatomen,

$R_4$ ein Wasserstoff- oder Halogenatom, eine Alkyl-, Amino-, Alkylamino-, Dialkylamino-, Alkanoylamino-, Alkoxycarbonylamino-, Alkylsulfonylamino-, Bis(alkylsulfonyl)-amino-, N-Alkyl-alkylsulfonylamino-, Cyano-, Alkylmercapto-, Alkylsulfinyl- oder Alkylsulfonylgruppe oder eine gegebenenfalls durch eine Alkyl-, Phenylalkyl-, 2-Hydroxyäthyl-, 3-Hydroxy-n-propyl-, 2-Hydroxy-n-propyl-, Alkylsulfonyl-, Cyanoalkyl-, Alkoxycarbonyl-, Hydroxycarbonylalkyl-, Alkoxycarbonylalkyl-, Trifluormethyl-, Difluormethyl- oder Trifluormethylsulfonylgruppe substituierte Hydroxygruppe,

$R_5$ ein Wasserstoff- oder Halogenatom, eine Alkyl-, Hydroxy-, Alkoxy-, Nitro-, Cyano- oder Trifluormethylgruppe oder

$R_4$ und $R_5$ zusammen eine Alkylendioxygruppe mit 1 oder 2 Kohlenstoffatomen und

$R_6$ ein Wasserstoff- oder Halogenatom, eine Alkyl- oder Alkoxygruppe, wobei alle vorstehend erwähnten Alkyl- oder Alkoxygruppen jeweils 1 bis 3 Kohlenstoffatome und die vorstehend erwähnten Alkanoylgruppen jeweils 2 oder 3 Kohlenstoffatome enthalten können, bedeuten, deren Enantiomere, deren Diastereomere und deren Säureadditionssalze.

2. Neue Naphthalin- und Indanderivate der Formel I gemäß Anspruch 1, in der

n die Zahl 1 oder 2,

A eine Carbonylgruppe und $R_7$ ein Wasserstoffatom oder

A eine Gruppe der Formel

$$\begin{array}{c} R_8 \\ | \\ -CH-, \end{array}$$

in welcher $R_8$ ein Wasserstoffatom oder eine Hydroxygruppe bedeutet, und $R_7$ ein Wasserstoffatom oder $R_7$ und $R_8$ zusammen eine weitere Bindung darstellen,

E die n-Propylengruppe,

G die Ethylen- oder n-Propylengruppe,

$R_1$ eine Methyl- oder Methoxygruppe,

$R_2$ ein Wasserstoffatom oder eine Methoxygruppe,

$R_3$ die Methylgruppe,

$R_4$ ein Wasserstoff-, Chlor- oder Bromatom, eine Hydroxy-, Methoxy-, Cyano-, Cyanomethoxy-, Hydroxycarbonylmethoxy-, Methoxycarbonylmethoxy-, Ethoxycarbonylmethoxy-, Methyl-, Amino-, Acetylamino-, Methoxycarbonylamino-, Methylsulfonyloxy-, Trifluormethylsulfonyloxy-, Benzyloxy-, Methylsulfonylamino- oder Bis(methylsulfonyl)-aminogruppe,

$R_5$ ein Wasserstoff-, Chlor- oder Bromatom, eine Methoxy- oder Nitrogruppe und

$R_6$ ein Wasserstoff-, Chlor- oder Bromatom bedeuten, deren Enantiomere, deren Diastereomere und deren Säureadditionssalze.

3. Neue Naphthalin- und Indanderivate der Formel I gemäß Anspruch 1, in der

$R_1$ bis $R_3$, A, E, G und n wie im Anspruch 2 definiert sind,

$R_4$ eine Methoxygruppe,

$R_5$ eine Methoxygruppe und

$R_6$ ein Wasserstoffatom bedeuten, deren Enantiomere, deren Diastereomere und deren Säureadditionssalze.

4. 6,7-Dimethoxy-2-[3-((2-(3,4-dimethoxyphenyl)-ethyl)-methylamino)-propyl]-1-oxo-1,2,3,4-tetrahydro-naphthalin und dessen Säureadditionssalze.

5. 6,7-Dimethoxy-2-[3-((2-(3,4-dimethoxyphenyl)-ethyl)-methylamino)-propyl]-1,2,3,4-tetrahydro-naphthalin und dessen Säureadditionssalze.

6. Physiologisch verträgliche Säureadditionssalze der Verbindungen gemäß den Ansprüchen 1 bis 5 mit anorganischen oder organischen Säuren.

7. Arzneimittel, enthaltend eine Verbindung der allgemeinen Formel I gemäß den Ansprüchen 1 bis 5 oder dessen physiologisch verträgliches Säureadditionssalz gemäß Anspruch 6 neben einem oder mehreren inerten Trägerstoffen und/oder Verdünnungsmitteln.

8. Arzneimittel gemäß Anspruch 7 geeignet zur Behandlung von Sinustachykardien und ischämischen Herzerkrankungen.

9. Verfahren zur Herstellung eines Arzneimittels gemäß den Ansprüchen 7 und 8, dadurch gekennzeichnet, daß auf nicht chemischem Wege eine Verbindung gemäß den Ansprüchen 1 bis 5 oder dessen physiologisch verträgliches Säureadditionssalz gemäß Anspruch 6 in einen oder mehreren inerten Trägerstoffen und/oder Verdünnungsmitteln eingearbeitet wird.

10. Verfahren zur Herstellung von neuen Naphthalin- und Indanderivaten gemäß den Ansprüchen 1 bis 6, dadurch gekennzeichnet, daß

a) zur Herstellung von Verbindungen der Formel I, in der $R_7$ und $R_8$ je ein Wasserstoffatom oder zusammen eine weitere Bindung darstellen, eine Verbindung der Formel

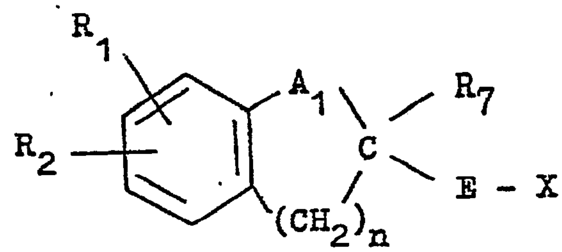

(II)

in der
$R_1$, $R_2$, n und E wie in den Ansprüchen 1 bis 5 definiert sind,

$A_1$ eine Gruppe der Formel

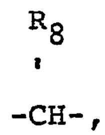

in welcher $R_8$ ein Wasserstoffatom bedeutet, und $R_7$ ein Wasserstoffatom oder $R_7$ und $R_8$ zusammen eine weitere Bindung darstellen, und

X eine nukleophile Austrittsgruppe wie ein Halogenatom oder eine Sulfonyloxygruppe darstellt, mit einem Amin der Formel

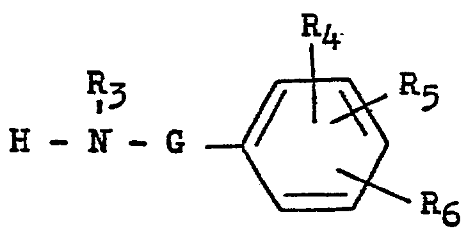

(III)

in der
$R_3$ bis $R_6$ und G wie in den Ansprüchen 1 bis 5 definiert sind, umgesetzt wird oder
b) eine Verbindung der Formel

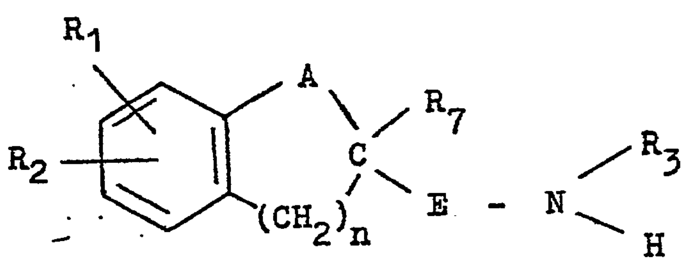

(IV)

in der
n, A, E, $R_1$ bis $R_3$ und $R_7$ wie in den Ansprüchen 1 bis 5 definiert sind, mit einer Verbindung der Formel

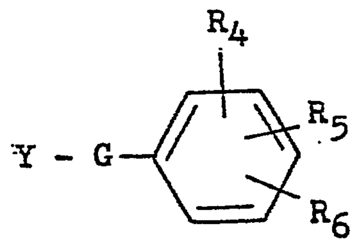

(V)

in der
$R_4$ bis $R_6$ und G wie in den Ansprüchen 1 bis 5 definiert sind und
Y eine nukleophile Austrittsgruppe wie ein Halogenatom oder eine Sulfonyloxygruppe darstellt, umgesetzt wird oder
c) zur Herstellung von Verbindungen der Formel I, in der A eine Gruppe der Formel

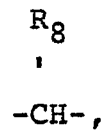

darstellt, in welcher $R_8$ ein Wasserstoffatom oder eine Hydroxygruppe bedeutet, eine Verbindung der Formel

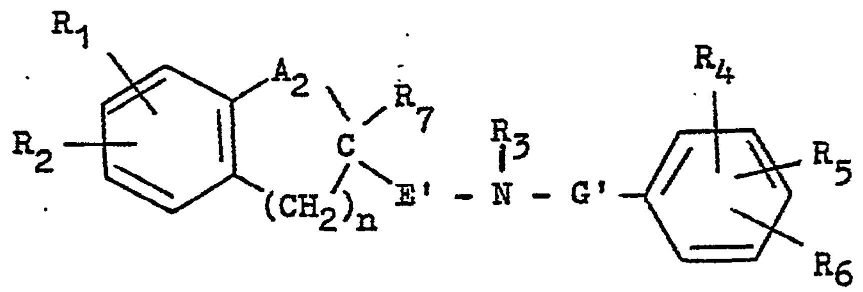

(VI)

in der
n und $R_1$ bis $R_6$ wie in den Ansprüchen 1 bis 5 definiert sind,

$A_2$ eine Carbonylgruppe und $R_7$ ein Wasserstoffatom oder

$A_2$ eine Gruppe der Formel

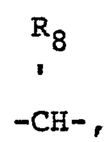

in welcher $R_8$ ein Wasserstoffatom oder eine Hydroxygruppe darstellt, und $R_7$ ein Wasserstoffatom,

E' die für E oder G' die für G in den Ansprüchen 1 bis 5 erwähnten Bedeutungen besitzt, wobei jedoch in den Resten E oder G eine zu der

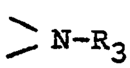

-Gruppe benachbarte Methylengruppe durch eine Carbonylgruppe ersetzt sein muß, reduziert wird oder
d) zur Herstellung von Verbindungen der Formel I, in der A die Carbonylgruppe darstellt, eine Verbindung der Formel

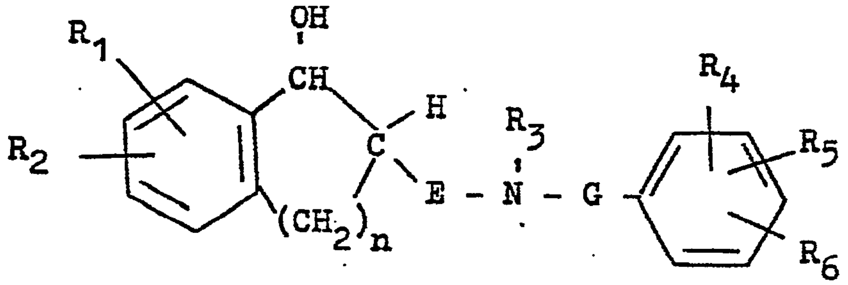

(VII)

in der
n, E, G und $R_1$ bis $R_6$ wie in den Ansprüchen 1 bis 5 definiert sind, oxidiert wird oder
e) zur Herstellung von Verbindungen der Formel I, in der
$R_4$ eine Alkylamino-, Dialkylamino-, Alkanoylamino-, Alkoxycarbonylamino-, Alkylsulfonylamino-, Bis(alkylsulfonyl)-amino-, N-Alkyl-alkylsulfonylamino-, Alkylmercapto-, Alkoxy-, Alkoxycarbonyloxy-, Hydroxycarbonylalkoxy-, Alkoxycarbonylalkoxy-, Phenylalkoxy-, Trifluormethoxy-, Drifluormethoxy-, Cyanoalkoxy-, Alkylsulfonyloxy- oder Trifluormethylsulfonyloxygruppe darstellt, eine Verbindung der Formel

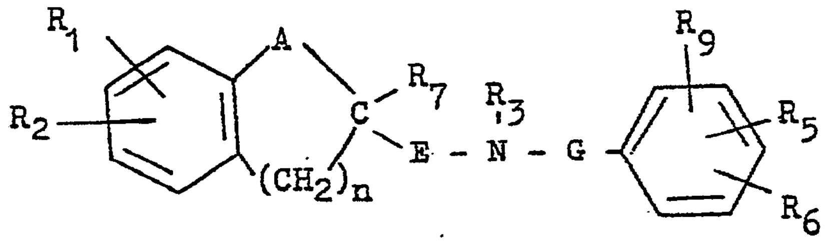

(VIII)

in der
$R_1$ bis $R_3$, $R_5$ bis $R_7$, A, E, G und n wie in den Ansprüchen 1 bis 5 definiert sind und
$R_9$ eine Hydroxy-, Amino- oder Alkylaminogruppe mit 1 bis 3 Kohlenstoffatomen darstellt, mit einer Verbindung der Formel
Z - $R_{10}$(IX)
in der
Z eine nukleophile Austrittsgruppe wie ein Halogenatom und $R_{10}$ eine Alkyl-, Alkanoyl-, Alkoxycarbonyl-, Hydroxycarbonylalkyl-, Alkoxycarbonylalkyl-, Alkylsulfonyl-, Phenylalkyl-, Trifluormethyl-, Difluormethyl- oder Cyanoalkylgruppe bedeuten, wobei die vorstehend erwähnten Alkyl- und Alkoxyteile jeweils 1 bis 3 Kohlenstoffatome bzw. der Alkanoylteil 2 oder 3 Kohlenstoffatome enthalten kann, umgesetzt wird oder
g) zur Herstellung von Verbindungen der Formel I, in der $R_7$ und $R_8$ zusammen eine weitere Bindung darstellen, eine Verbindung der Formel

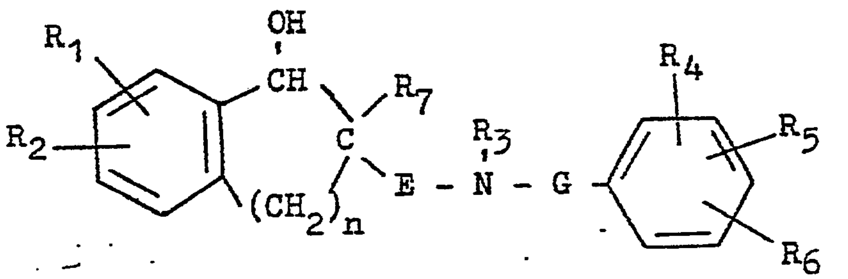

(X)

in der
n, E, G und $R_1$ bis $R_7$ wie in den Ansprüchen 1 bis 5 definiert sind, dehydratisiert wird,

wobei erforderlichenfalls eine bei den Umsetzungen a) bis g) zum Schutze von reaktiven Gruppen verwendete Schutzgruppe anschließend abgespalten wird

und gewünschtenfalls anschließend eine so erhaltene Verbindung der Formel I, in der $R_4$ eine Benzyloxygruppe darstellt, mittels Entbenzylierung in die entsprechende Hydroxyverbindung übergeführt wird und/oder

eine so erhaltene Verbindung der Formel I, sofern sie mindestens ein chirales Zentrum enthält, in ihre Diastereomeren oder in ihre Enantiomeren aufgetrennt wird und/oder

eine so erhaltene Verbindung der Formel I in ihre Säureadditionssalze, insbesondere in ihre physiologisch verträglichen Säureadditionssalze mit anorganischen oder organischen Säuren übergeführt wird.

**Claims**

1. New naphthalene and indan derivatives of formula

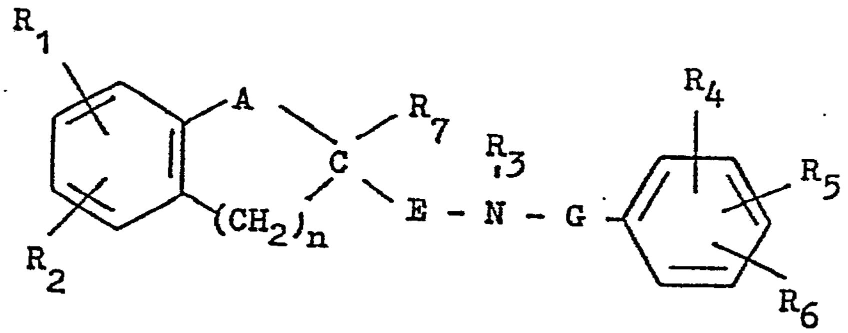

(I)

wherein
n represents the number 1 or 2,
A represents a carbonyl group and $R_7$ represents a hydrogen atom, or
A represents a group of formula

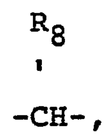

wherein $R_8$ represents a hydrogen atom or a hydroxy, alkanoyloxy or alkoxycarbonyloxy group, and R7 represents a hydrogen atom or $R^{\cdot}_7$and $R_8$ together form another bond,
E represents a straight-chained alkylene group with 3 or 4 carbon atoms optionally substituted by an alkyl group,
G represents a straight chained alkylene group with 2 to 5 carbon atoms optionally substituted by an alkyl group,
$R_1$ represents a hydrogen or halogen atom or a trifluoromethyl, nitro, amino, alkylamino, dialkylamino, alkyl, hydroxy, alkoxy of phenylalkoxy group,
$R_2$ represents a hydrogen or halogen atom or a hydroxy, alkoxy, phenylalkoxy or alkyl group, or
$R_1$ and $R_2$ together represent an alkylenedioxy group with 1 or 2 carbon atoms,
$R_3$ represents a hydrogen atom, an alkyl group or an alkenyl group with 3 to 5 carbon atoms,
$R_4$ represents a hydrogen or halogen atom, an alkyl, amino, alkylamino, dialkylamino, alkanoylamino, alkoxycarbonylamino, alkylsulphonylamino, bis(alkyl-sulphonyl)-amino, N-alkyl-alkylsulphonylamino, cyano, alkylmercapto, alkylsulphinyl or alkylsulphonyl group, or a hydroxy group optionally substituted by an alkyl, phenylalkyl, 2-hydroxyethyl, 3-hydroxy-n-propyl, 2-hydroxy-n-propyl, alkylsulphonyl, cyanoalkyl, alkoxycarbonyl, hydroxycarbonylalkyl, alkoxycarbonylalkyl, trifluoromethyl, difluoromethyl or trifluoromethyl-sulphonyl group,
$R_5$ represents a hydrogen or halogen atom or an alkyl, hydroxy, alkoxy, nitro, cyano or trifluoromethyl group, or
$R_4$ and $R_5$ together represent an alkylenedioxy group with 1 or 2 carbon atoms, and
$R_6$ represents a hydrogen or halogen atom or an alkyl or alkoxy group,
whilst all the above-mentioned alkyl or alkoxy groups may each contain 1 to 3 carbon atoms and the above-mentioned alkanoyl groups may each contain 2 or 3 carbon atoms,
the enantiomers, diastereomers and acid addition salts thereof

2. New naphthalene and indan derivatives of formula I as claimed in claim 1, wherein
n represents the number 1 or 2,
A represents a carbonyl group and $R_7$ represents a hydrogen atom, or
A represents a group of formula

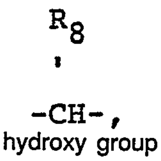

wherein $R_8$ represents a hydrogen atom or a hydroxy group and $R_7$ represents a hydrogen atom or $R_7$ and $R_8$ together represent another bond,
E represents the n-propylene group,
G represents the ethylene or n-propylene group,
$R_1$ represents a methyl or methoxy group,
$R_2$ represents a hydrogen atom or a methoxy group,
$R_3$ represents a methyl group,
$R_4$ represents a hydrogen, chlorine or bromine atom, a hydroxy, methoxy, cyano, cyanomethoxy, hydroxycarbonyl-methoxy, methoxycarbonylmethoxy, ethoxycarbonylmethoxy, methyl, amino, acetylamino, methoxycarbonyl-amino, methylsulphonyloxy, trifluoromethyl-sulphonyloxy, benzyloxy, methylsulphonylamino or bis(methylsulphonyl)amino group,
$R_5$ represents a hydrogen, chlorine or bromine atom or a methoxy or nitro group, and
$R_6$ represents a hydrogen, chlorine or bromine atom,
the enantiomers, diastereomers and acid addition salts thereof.

3. New naphthalene and indan derivatives of formula I as claimed in claim 1, wherein
$R_1$ to $R_3$, A, E, G and n are defined as in claim 2,
$R_4$ represents a methoxy group,
$R_5$ represents a methoxy group, and
$R_6$ represents a hydrogen atom,
the enantiomers, diastereomers and acid addition salts thereof.

4. 6,7-Dimethoxy-2-[3-((2-(3,4-dimethoxyphenyl)-ethyl)-methylamino)-propyl]-1-oxo-1,2,3,4-tetrahydronaphthalene and the acid addition salts thereof.

5. 6,7-Dimethoxy-2-[3-((2-(3,4-diemthoxyphenyl)-ethyl)-methylamino)-propyl]-1,2,3,4-tetrahydronaphthalene and the acid addition salts thereof.

6. Physiologically acceptable acid addition salts of the compounds as claimed in claims 1 to 5 with inorganic or organic acids.

7. Pharmaceutical compositions containing a compound of general formula I as claimed in claims 1 to 5 or a physiologically acceptable acid addition salt thereof as claimed in claim 6 together with one or more inert carriers and/or diluents.

8. Pharmaceutical compositions as claimed in claim 7 suitable for the treatment of sins tachycardia and ischaemic heart disease.

9. Process for preparing a pharmaceutical composition as claimed in claims 7 and 8, characterised in that a compound as claimed in claim 1 to 5 or a physiologically acceptable acid addition salt thereof as claimed in claim 6 is incorporated, by a non-chemical method, in one or more inert carriers and/or diluents.

10. Process for preparing new naphthalene and indan derivatives as claimed in claims 1 to 6, characterised in that

a) in order to prepare compounds of formula I wherein $R_7$ and $R_8$ each represent a hydrogen atom or together represent another bond:
a compound of formula

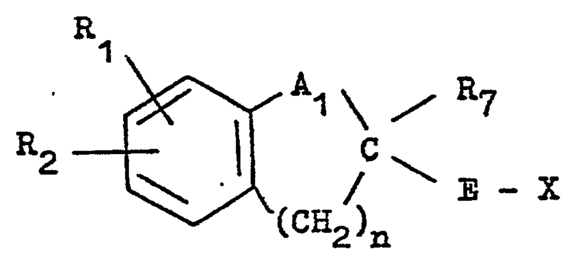

(II)

wherein
$R_1$, $R_2$, n and E are defined as in claims 1 to 5,
$A_1$ represents a group of formula

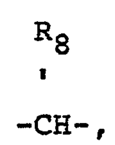

wherein $R_8$ represents a hydrogen atom, and $R_7$ represents a hydrogen atom or $R_7$ and $R_8$ together represent another bond, and
X represents a nucleophilically exchangeable group such as a halogen atom or a sulphonyloxy group, is reacted with an amine of formula

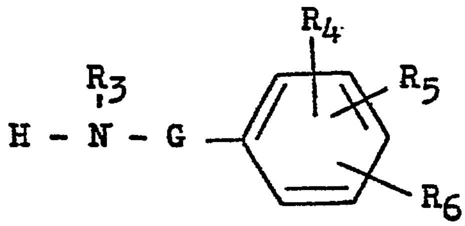

(III)

wherein
$R_3$ to $R_6$ and G are defined as in claims 1 to 5, or
b) a compound of formula

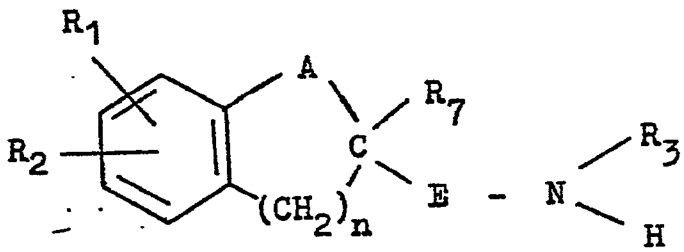

(IV)

wherein
n, A, E, $R_1$ to $R_3$ and $R_7$ are defined as in claims 1 to 5, is reacted with a compound of formula

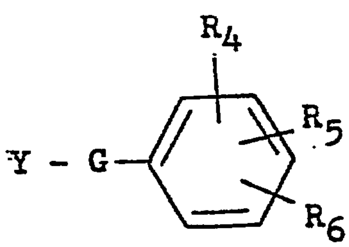

(V)

wherein
$R_4$ to $R_6$ and G are defined as in claims 1 to 5, and
Y represents a nucleophilically exchangeable group such as a halogen atom or a sulphonyloxy group, or
c) in order to prepare compounds of formula I wherein
A represents a group of formula

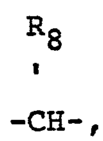

wherein $R_8$ represents a hydrogen atom or a hydroxy group:
a compound of formula

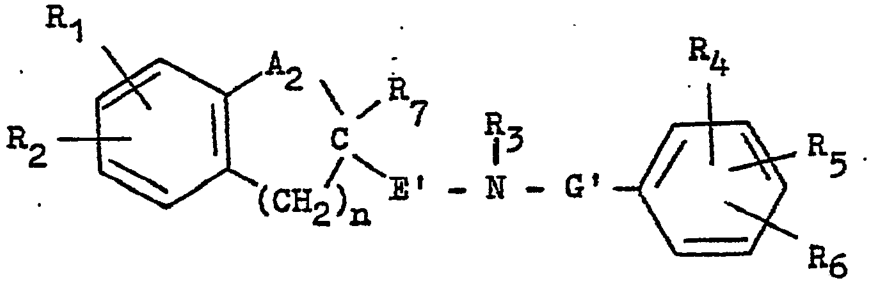 (VI)

wherein
n and $R_1$ to $R_6$ are defined as in claims 1 to 5,
$A_2$ represents a carbonyl group and $R_7$ represents a hydrogen atom, or
$A_2$ represents a group of formula

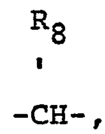

wherein $R_8$ represents a hydrogen atom or a hydroxy group, and $R_7$ represents a hydrogen atom,
E′ has the meanings given in claims 1 to 5 for E, or G′ has the meanings given for G in claims 1 to 5, but in the group E or G a methylene group adjacent to the

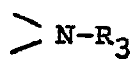

group must be replaced by a carbonyl group, or
d) in order to prepare compounds of formula I wherein
A represents the carbonyl group:
a compound of formula

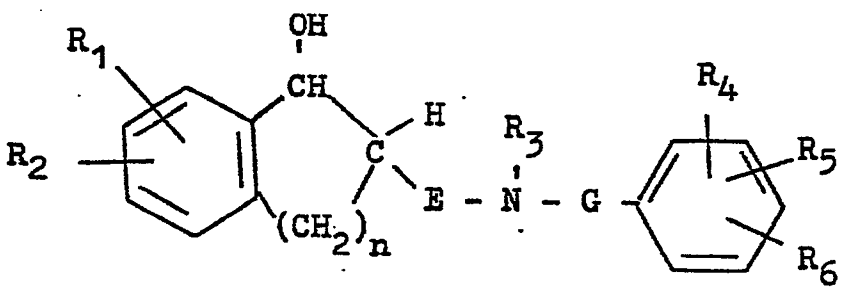 (VII)

wherein
n, E, G and $R_1$ to $R_6$ are defined as in claims 1 to 5, is oxidised, or
e) in order to prepare compounds of formula I wherein $R_4$ represents an alkylamino, dialkylamino, alkanoylamino, alkoxycarbonylamino, alkylsulphonylamino, bis(alkylsulphonyl)amino, N-alkyl-alkylsulphonylamino, alkylmercapto, alkoxy, alkoxycarbonyloxy, hydroxycarbonylalkoxy, alkoxycarbonylalkoxy, phenylalkoxy, trifluoromethoxy, difluoromethoxy, cyanoalkoxy, alkylsulphonyloxy or trifluoromethylsulphonyloxy group:
a compound of formula

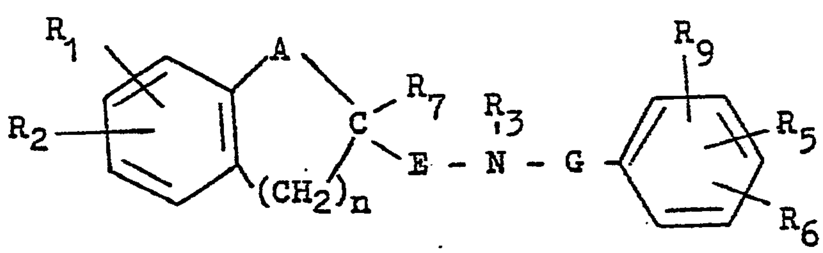 (VIII)

wherein
$R_1$ to $R_3$, $R_5$ to $R_7$, A, E, G and n are defined as in claims 1 to 5, and
$R_9$ represents a hydroxy, amino or alkylamino group with 1 to 3 carbon atoms,
is reacted with a compound of formula

$Z - R_{10}$ (IX)

wherein
Z represents a nucleophilically exchangeable group such as a halogen atom, and
$R_{10}$ represents an alkyl, alkanoyl, alkoxycarbonyl, hydroxycarbonylalkyl, alkoxycarbonylalkyl, alkylsulphonyl, phenylalkyl, trifluoromethyl, difluoromethyl or cyanoalkyl group, whilst the above-mentioned alkyl and alkoxy moieties may each contain from 1 to 3 carbon atoms and the alkanoyl moiety may contain 2 or 3 carbon atoms, or
g) in order to prepare compounds of formula I wherein $R_7$ and $R_8$ together represent another bond:
a compound of formula

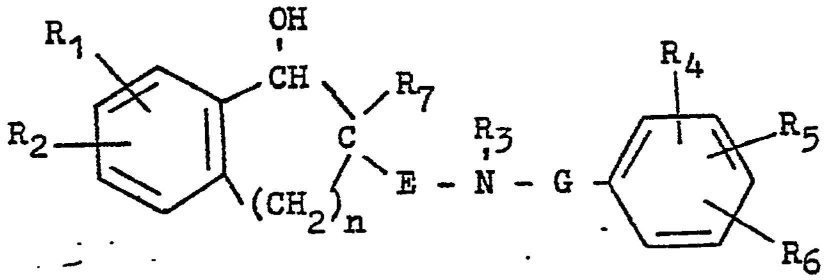
(X)

wherein
n, E, G and $R_1$ to $R_7$ are defined as in claims 1 to 5, is dehydrated,
whilst if necessary any protecting group used in the reactions a) to g) in order to protect reactive groups is subsequently split off,
and subsequently, if desired, a compound of formula I obtained wherein $R_4$ represents a benzyloxy group is converted by debenzylation into the corresponding hydroxy compound, and/or
a compound of formula I thus obtained, if it contains at least one chiral centre, is resolved into its diastereomers or its enantiomers, and/or
a compound of formula I thus obtained is converted into its acid addition salts, particularly its physiologically acceptable addition salts with inorganic or organic acids.

## Revendications

1. Nouveaux dérivés de naphtalène et de l'indane de formule

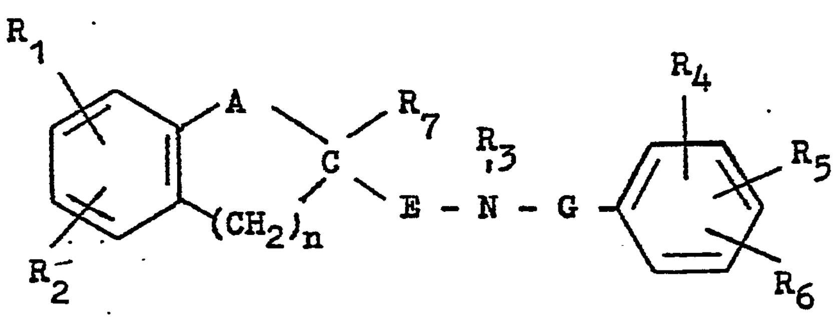
(I)

dans laquelle
n représente le nombre 1 ou 2,
A représente un groupe carbonyle et $R_7$ un atome d'hydrogène ou
A représente un groupe de formule

$$\begin{array}{c} R_8 \\ | \\ -CH-, \end{array}$$

dans laquelle $R_8$ représente un atome d'hydrogène, un groupe hydroxy, alcanoyloxy ou alcoxycarbonyloxy, et $R_7$ représente un atome d'hydrogène ou $R_7$ et $R_8$ représentent ensemble une liaison supplémentaire,

E représente un groupe alcoylène avec 3 ou 4 atomes de carbone, rectiligne, éventuellement substitué par un groupe alcoyle,

G représente un groupe alcoylène avec 2 à 5 atomes de carbone, rectiligne, éventuellement substitué par un groupe alcoyle,

$R_1$ représente un atome d'hydrogène ou d'halogène, un groupe trifluorométhyle, nitro, amino, alcoylamino, dialcoylamino, alcoyle, hydroxy, alcoxy ou phénylalcoxy, $R_2$ représente un atome d'hydrogène ou un atome d'halogène, un groupe hydroxy, alcoxy, phénylalcoxy ou alcoyle, ou

$R_1$ et $R_2$ représentent ensemble un groupe alcoylènedioxy avec 1 ou 2 atomes de carbone,

$R_3$ représente un atome d'hydrogène, un groupe alcoyle ou un groupe alcényle avec 3 à 5 atomes de carbone,

$R_4$ représente un atome d'hydrogène ou d'halogène, un groupe alcoyle, amino, alcoylamino, dialcoylamino, alcanoylamino, alcoxycarbonylamino, alcoylsulfonylamino, bis(alcoylsulfonyl)amino, N-alcoyl-alcoylsulfonylamino, cyano, alcoylmercapto, alcoylsulfinyle ou alcoylsulfonyle, ou un groupe hydroxy éventuellement substitué par un groupe alcoyle, phénylalcoyle, 2-hydroxyéthyle, 3-hydroxy-n-propyle, 2-hydroxy-n-propyle, alcoylsulfonyle, cyanoalcoyle, alcoxycarbonyle, hydroxycarbonylalcoyle, alcoxycarbonylalcoyle, trifluorométhyle, difluorométhyle ou trifluorométhylsulfonyle,

$R_5$ représente un atome d'hydrogène ou d'halogène, un groupe alcoyle, hydroxy, alcoxy, nitro, cyano ou trifluorométhyle, ou

$R_4$ et $R_5$ représentent ensemble un groupe alcoylènedioxy avec 1 ou 2 atomes de carbone et

$R_6$ représente un atome d'hydrogène ou d'halogène, un groupe alcoyle ou alcoxy, tous les groupes alcoyle ou alcoxy mentionnés plus haut pouvant contenir chacun 1 à 3 atomes de carbone et les groupes alcanoyle mentionnés plus haut pouvant contenir chacun 2 ou 3 atomes de carbone, leurs énantiomères, leurs diastéréoisomères et leurs sels d'addition d'acides.

2. Nouveaux dérivés du naphtalène et de l'indane de formule I selon la revendication 1, dans laquelle n représente le nombre 1 ou 2,

A représente un groupe carbonyle et $R_7$ un atome d'hydrogène ou

A représente un groupe de formule

$$\begin{array}{c} R_8 \\ | \\ -CH-, \end{array}$$

dans laquelle $R_8$ représente un atome d'hydrogène ou un groupe hydroxy, et

$R_7$ représente un atome d'hydrogène ou $R_7$ et $R_8$ représentent ensemble une liaison supplémantaire,

E représente le groupe n-propylène,

G représente le groupe éthylène ou n-propylène,

$R_1$ représente un groupe méthyle ou méthoxy,

$R_2$ représente un atome d'hydrogène ou un groupe méthoxy,

$R_3$ représente le groupe méthyle,

$R_4$ représente un atome d'hydrogène, de chlore ou de brome, un groupe hydroxy, méthoxy, cyano, cyanométhoxy, hydroxycarbonylméthoxy, méthoxycarbonylméthoxy, éthoxycarbonylméthoxy, méthyle, amino, acétylamino, méthoxycarbonylamino, méthylsulfonyloxy, trifluorométhylsulfonyloxy, benzyloxy, méthylsulfonylamino, ou bis(méthylsulfonyl)amino,

$R_5$ représente un atome d'hydrogène, de chlore ou de brome, un groupe méthoxy ou nitro et

$R_6$ représente un atome d'hydrogène, de chlore ou de brome, leurs énantiomères, leurs diastéréoisomères et leurs sels d'addition d'acides.

3. Nouveaux dérivés de naphtalène et de l'indane de formule I selon la revendication 1, dans laquelle $R_1$ à $R_3$, A, E, G et n sont définis comme dans la revendication 2,

$R_4$ représente un groupe méthoxy,

$R_5$ représente un groupe méthoxy, et

$R_6$ représente un atome d'hydrogène, leurs énantiomères, leurs diastéréoisomères et leurs sels d'addition d'acides.

4. Le 6,7-diméthoxy-2-[3-((2-(3,4-diméthoxyphényl)-éthyl)-méthylamino)-propyl]-1-oxo-1,2,3,4-tétrahydronaphtalène et ses sels d'addition d'acides.

5. Le 6,7-diméthoxy-2-[3-((2-(3,4-diméthoxyphényl)-éthyl)-méthylamino)-propyl]-1,2,3,4-tétrahydronaphtalène et ses sels d'addition d'acides.

6. Sels d'addition d'acides physiologiquement supportables des composés selon les revendications 1 à 5 avec des acides non organiques ou organiques.

7. Médicament contenant un composé de fomrule générale I selon les revendicaitons 1 à 5 ou son sel d'addition d'acide physiologiquement supportable selon la revendication 6, conjointement à un ou plusieurs excipients et/ou diluants inertes.

8. Médicament selon la revendication 7 convenant au traitement des tachycardies sinusales et des maladies cardiaques ischémiques.

9. Procédé pour la fabrication d'un médicament selon les revendications 7 et 8, caractérisé en ce que, par voie non chimique, on introduit un composé selon les revendications 1 à 5 ou son sel d'addition d'acide physiologiquement supportable selon la revendication 6, dans un ou plusieurs excipients et/ou diluants inertes.

10. Procédé pour la fabrication des nouveaux dérivés de naphtalène et de l'indane selon les revendications 1 à 6, caractérisé en ce que
a) pour la fabrication des composés de formule I dans laquelle $R_7$ et $R_8$ représentent chacun un atome d'hydrogène ou représentent ensemble une liaison supplémentaire, on fait réagir un composé de formule

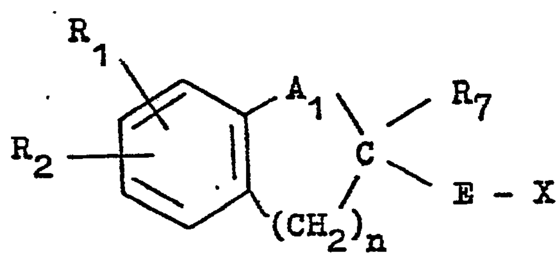

(II)

dans laquelle
$R_1$, $R_2$, n et E sont définis comme dans les revendications 1 à 5,
$A_1$ représente un groupe de formule

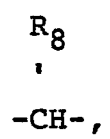

dans laquelle
$R_8$ représente un atome d'hydrogène, et $R_7$ représente un atome d'hydrogène ou $R_7$ et $R_8$ représentent ensemble une liaison supplémentaire, et
X représente un groupe partant nucléophile tel qu'un atome d'halogène ou un groupe sulfonyloxy, avec une amine de formule

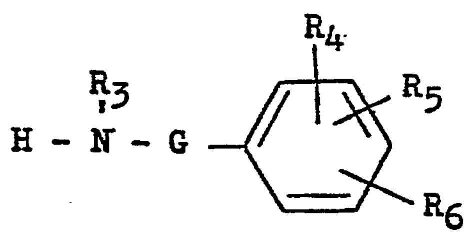

(III)

dans laquelle
$R_3$ à $R_6$ et G sont définis comme dans les revendications 1 à 5 ou
b) on fait réagir un composé de formule

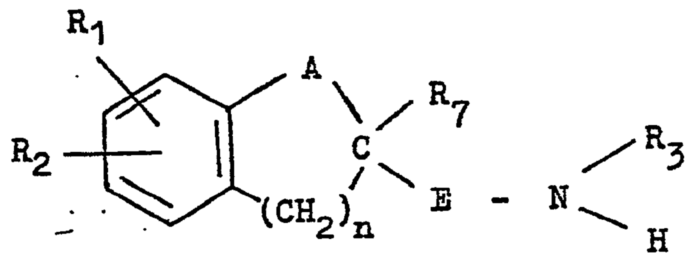

(IV)

dans laquelle
n, A, E, $R_1$ à $R_3$ et $R_7$ sont définis comme dans les revendications 1 à 5, avec un composé de formule

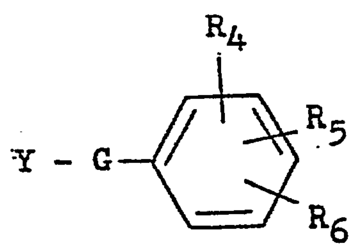

(V)

dans laquelle
$R_4$ à $R_6$ et G sont définis comme dans les revendications 1 à 5 et Y représente un groupe partant nucléophile tel qu'un atome d'halogène ou un groupe sulfonyloxy ou
c) pour la fabrication des composés de formule I dans laquelle A représente un groupe de formule

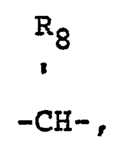

dans laquelle $R_8$ représente un atome d'hydrogène ou un groupe hydroxy, on réduit un composé de formule

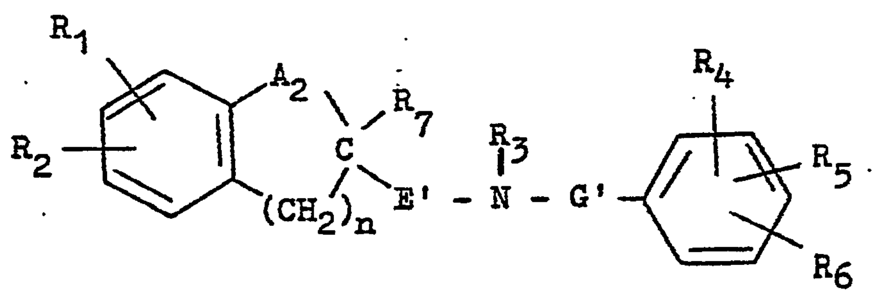

(VI)

dans laquelle
n et $R_1$ à $R_6$ sont définis comme dans les revendications 1 à 5,
$A_2$ représente un groupe carbonyle et $R_7$ un atome d'hydrogène ou
$A_2$ représente un groupe de formule

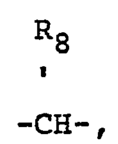

dans
$R_8$ représente un atome d'hydrogène ou groupe hydroxy, et
$R_7$ représente un atome d'hydrogène,
E′ possède les significations mentionnées dans les revendications 1 à 5 pour E ou G′ possède les significations mentionnées dans les revendications 1 à 5 pour G, toutefois dans les radicaux E ou G un groupe

méthylène voisin du groupe N–$R_3$ devant être remplacé par un groupe carbonyle ou
d) pour la fabrication des composés de formule I dans laquelle A représente le groupe carbonyle, on oxyde un composé de formule

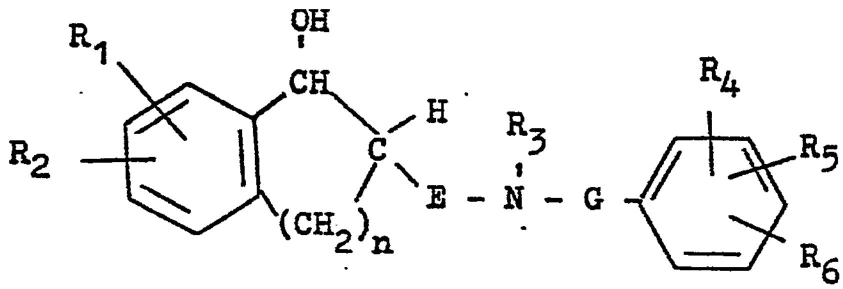
(VII)

dans laquelle
n, E, G et $R_1$ à $R_6$ sont définis comme dans les revendications 1 à 5 ou
e) pour la fabrication des composés de formule I dans laquelle $R_4$ représente un groupe alcoylamino, dialcoylamino, alcanoylamino, alcoxycarbonylamino, alcoylsulfonylamino, bis(alcoylsulfonyl)amino, N-alcoyl-alcoylsulfonylamino, alcoylmercapto, alcoxy, alcoxycarbonyloxy, hydroxycarbonylalcoxy, alcoxycarbonylalcoxy, phénylalcoxy, trifluorométhoxy, difluorométhoxy, cyanoalcoxy, alcoylsulfonyloxy ou trifluorométhylsulfonyloxy, on fait réagir un composé de formule

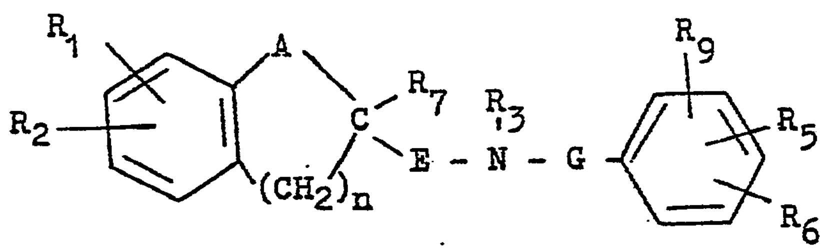
(VIII)

dans laquelle
$R_1$ à $R_3$, $R_5$ à $R_7$, A, E, G et n sont définis comme dans les revendications 1 à 5, et
$R_9$ représente un groupe hydroxy, amino ou alcoylamino avec 1 à 3 atomes de carbone, avec un composé de formule

**Z - R>! )(IX)**

dans laquelle
Z représente un groupe partant nucléophile tel qu'un atome d'halogène et
$R_{10}$ représente un groupe alcoyle, alcanoyle, alcoxycarbonyle, hydroxycarbonylalcoyle, alcoxycarbonylalcoyle, alcoylsulfonyle, phénylalcoyle, trifluorométhyle, difluorométhyle ou cyanoalcoyle, les parties alcoyle et alcoxy mentionnées plus haut pouvant contenir chacune 1 à 3 atomes de carbone ou respectivement la partie alcanoyle 2 à 3 atomes de carbone ou
g) pour la fabrication des composés de formule I dans laquelle $R_7$ et $R_8$ représentent ensemble une liaison supplémentaire, on déshydrate un composé de formule

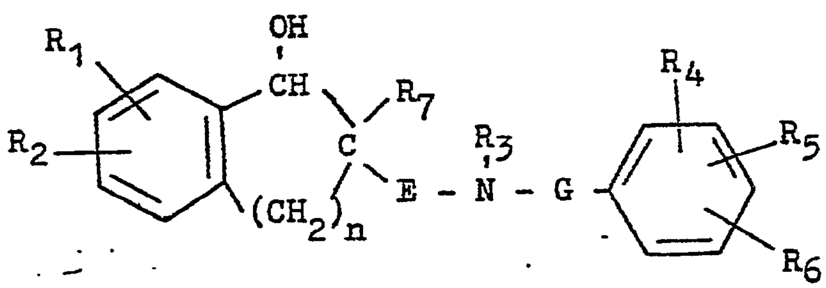
(X)

dans laquelle
n, E, G et $R_1$ à $R_7$ sont définis comme dans les revendications 1 à 5,
si nécessaire un groupe protecteur utilisé pour la protection des groupes réactifs dans les réactions a)